(19) Europäisches Patentamt
European Patent Office
Office européen des brevets

(11) Veröffentlichungsnummer: **0 260 621 A2**

(12) # EUROPÄISCHE PATENTANMELDUNG

(21) Anmeldenummer: **87113342.7**

(22) Anmeldetag: **11.09.87**

(51) Int. Cl.⁴: **C07D 239/54**, C07D 239/36, A01N 43/54

(30) Priorität: **18.09.86 CH 3741/86**
**24.07.87 CH 2828/87**

(43) Veröffentlichungstag der Anmeldung:
**23.03.88 Patentblatt 88/12**

(84) Benannte Vertragsstaaten:
**CH DE ES FR GB IT LI NL**

(71) Anmelder: **F. HOFFMANN-LA ROCHE & CO. Aktiengesellschaft**

**CH-4002 Basel(CH)**

(72) Erfinder: **Wenger, Jean, Dr.**
**Brunnenwiesenstrasse 1**
**CH-8610 Uster(CH)**
Erfinder: **Winternitz, Paul, Dr.**
**Am Pfisterhölzli 50**
**CH-8606 Greifensee(CH)**

(74) Vertreter: **Lederer, Franz, Dr. et al**
**Patentanwält Dr. Franz Lederer Lucile Grahnstrasse 22**
**D-8000 München 80(DE)**

(54) **3-Aryluracil-enoläther und deren Verwendung zur Unkrautbekämpfung.**

(57) Die Erfindung betrifft neue $C_{1-4}$-Alkyl-, $C_{2-4}$-Alkenyl-und $C_{3\ \text{oder}\ 4}$ -Alkinyl-enoläther von 3-Aryluracilen der Formel

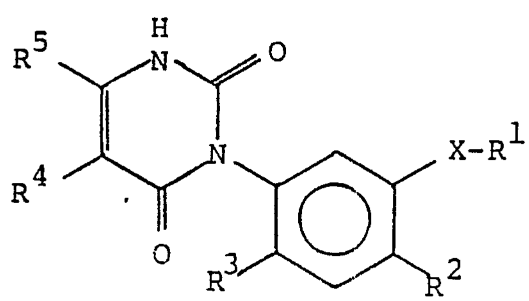

I'

worin $R^1$, $R^2$, $R^3$, $R^4$, $R^5$ und X die in der Beschreibung angegebenen Bedeutungen besitzen, deren Herstellung, Unkrautbekämpfungsmittel, die solche Enoläther als Wirkstoffe enthalten und die Verwendung der Wirkstoffe bzw. Mittel zur Unkrautbekämpfung. Die Erfindung betrifft ebenfalls gewisse, zum Teil herbizid wirksame Ausgangsmaterialien und solche Wirkstoffe enthaltende Unkrautbekämpfungsmittel.

EP 0 260 621 A2

## 3-Aryluracil-enoläther und deren Verwendung zur Unkrautbekämpfung

Die vorliegende Erfindung betrifft heterocyclische Verbindungen, und zwar $C_{1-4}$-Alkyl-, $C_{2-4}$-Alkenyl-und $C_{3\ oder\ 4}$-Alkinyl-enoläther von 3-Aryluracilen der allgemeinen Formel

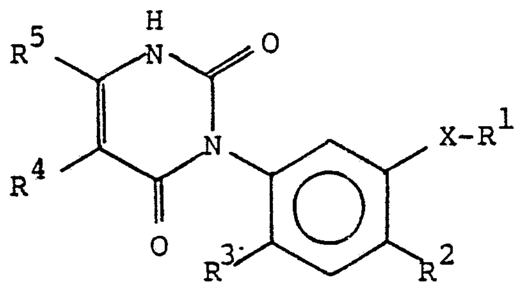

I'

worin

$R^1$ $C_{1-8}$-Alkyl, $C_{2-6}$-Alkenyl, $C_{2-6}$-Alkinyl, $C_{2-8}$ -Alkoxyalkyl oder eine Gruppe

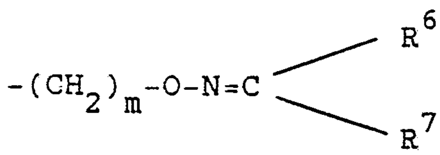

$R^2$ Halogen oder Cyano,
$R^3$ Wasserstoff oder Halogen,
$R^4$ Wasserstoff, Fluor oder $C_{1-4}$-Alkyl,
$R^5$ $C_{1-4}$-Alkyl oder $C_{1-4}$-Halogenalkyl,

oder

$R^4$ und $R^5$ zusammen Tri-oder Tetramethylen,
$R^6$ und $R^7$ unabhängig voneinander $C_{1-4}$-Alkyl,
m 1 oder 2

und

X O, O-C(O), O-C(O)-O oder C(O)-O bedeuten.

Die erfindungsgemässen Enoläther umfassen also die Verbindungen der Formeln

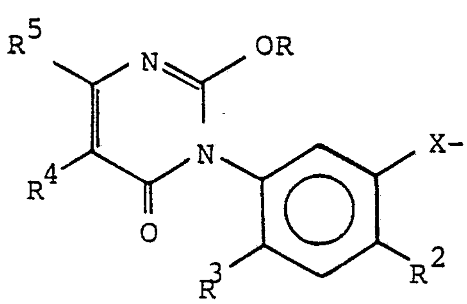

und

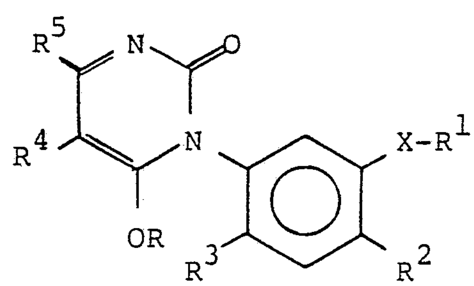

Ia

Ib

worin $R^1$-$R^5$ und X die oben angegebenen Bedeutungen besitzen und R $C_{1-4}$-Alkyl, $C_{2-4}$-Alkenyl oder $C_{3\ oder\ 4}$-Alkinyl bedeutet.

Die erfindungsgemässen Enoläther sind herbizid wirksam und eignen sich als Wirkstoffe von Unkrautbekämpfungsmitteln. Somit umfasst die Erfindung auch Unkrautbekämpfungsmittel, welche erfindungsgemässe Enoläther als Wirkstoffe enthalten, Verfahren zur Herstellung dieser Verbindungen sowie die Verwendung solcher Enoläther bzw. Mittel zur Bekämpfung von Unkräutern.

In den obigen Definitionen der Reste $R^2$, $R^3$ und $R^5$ umfasst "Halogen" als solches bzw. als Teil der Halogenalkylgruppe Fluor, Chlor, Brom und Jod. Die Halogenalkyl gruppe ($R^5$) kann ein oder mehrere gleiche oder verschiedene Halogenatome aufweisen, wobei als Beispiel eines mehrfach halogenierten Alkylrestes Trifluormethyl genannt sei. Die Alkyl-, Alkenyl-und Alkinylreste (R, $R^1$, $R^4$, $R^5$, $R^6$, $R^7$) können geradkettig oder verzweigt sein, wobei dies auch für den bzw. jeden Alkylteil der Alkoxalkyl-und Halogenalkylgruppen ($R^1$, $R^5$) gilt.

Eine Untergruppe der erfindungsgemässen Enoläther besteht aus den $C_{1-4}$-Alkyl-, $C_{3\ \text{oder}\ 4}$-Alkenyl-und $C_{3\ \text{oder}\ 4}$-Alkinyl-enoläthern von denjenigen 3-Aryluracilen der Formel I', in denen $R^1$ $C_{1-6}$-Alkyl, $C_{3-6}$-Alkenyl, $C_{3-6}$-Alkinyl, $C_{2-7}$-Alkoxyalkyl oder eine Gruppe

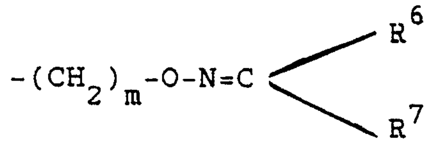

und $R^2$ Halogen bedeuten, und $R^3$, $R^4$, $R^5$, $R^6$, $R^7$, m und X die oben angegebenen Bedeutungen besitzen.

Unabhängig voneinander bedeuten $R^2$ vorzugsweise Chlor oder Brom, $R^3$ vorzugsweise Fluor und $R^5$ vorzugsweise $C_{1-4}$-Fluoralkyl, insbesondere Trifluormethyl oder Pentafluoräthyl.

Besonders bevorzugte einzelne erfindungsgemässe Verbindungen sind:

2-Chlor-4-fluor-5-[2-methoxy-6-oxo-4-pentafluoräthyl-1(6H)-pyrimidinyl]-benzoesäure-isopropylester,
2-Chlor-4-fluor-5-[5-fluor-2-methoxy-6-oxo-4-pentafluoräthyl-1(6H)-pyrimidinyl]-benzoesäure-isopropylester,
1-(4-Chlor-2-fluor-5-isopropoxyphenyl)-2-methoxy-4-trifluormethyl-6(1H)-pyrimidinon,
1-(4-Chlor-2-fluor-5-isopropoxyphenyl)-2-methoxy-4-pentafluoräthyl-6(1H)-pyrimidinon,
1-(4-Chlor-2-fluor-5-isopropoxyphenyl)-5-fluor-2-methoxy-4-pentafluoräthyl-6(1H)-pyrimidinon,
2-Chlor-4-fluor-5-[2-methoxy-6-oxo-4-trifluormethyl-1(6H)-pyrimidinyl]-benzoesäure-isopropylester,
2-Chlor-4-fluor-5-[5-fluor-2-methoxy-6-oxo-4-trifluormethyl-1(6H)-pyrimidinyl]-benzoesäure-isopropylester,
1-(4-Chlor-2-fluor-5-propargyloxyphenyl)-2-methoxy-4-trifluormethyl-6(1H)-pyrimidinon,
2-Chlor-4-fluor-5-[2-methoxy-6-oxo-4-pentafluoräthyl-1(6H)-pyrimidinyl]-benzoesäure-methylester,
2-Chlor-4-fluor-5-[2-methoxy-6-oxo-4-pentafluoräthyl-1(6H)-pyrimidinyl]-benzoesäure-äthylester,
2-Chlor-4-fluor-5-[2-methoxy-6-oxo-4-pentafluoräthyl-1(6H)-pyrimidinyl]-benzoesäure-(n-propyl)ester,
2-Chlor-4-fluor-5-[2-methoxy-6-oxo-4-pentafluoräthyl-1(6H)-pyrimidinyl]-benzoesäure-(n-butyl)ester und
2-Chlor-4-fluor-5-[2-methoxy-6-oxo-4-pentafluoräthyl-1(6H)-pyrimidinyl]-benzoesäure-2-methoxyäthylester.

Weitere Vertreter von erfindungsgemässen Verbindungen sind diejenigen der Formeln

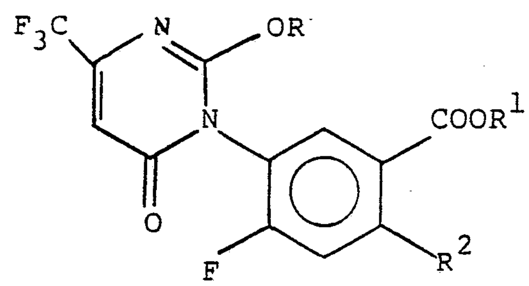

$Ia^1$

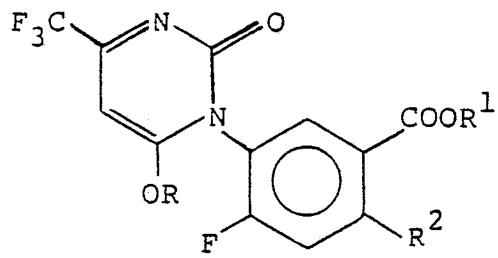

$Ib^1$

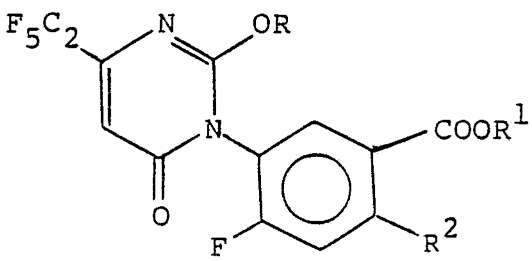

$Ia^2$

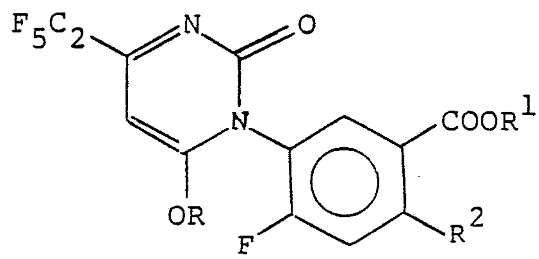

$Ib^2$

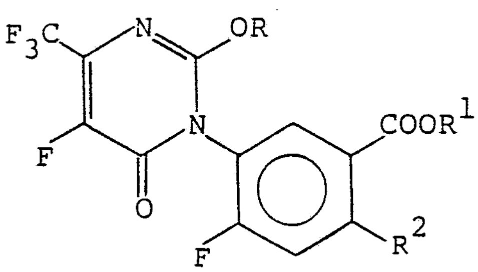

$Ia^3$

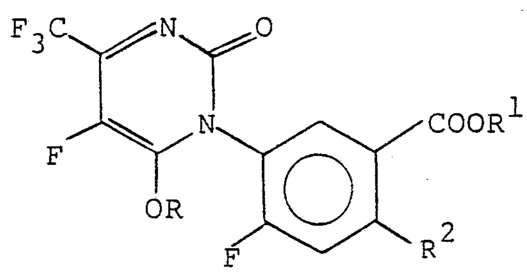

$Ib^3$

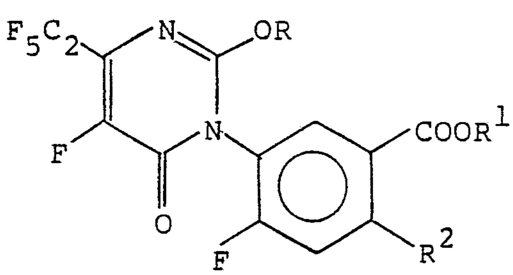

$Ia^4$

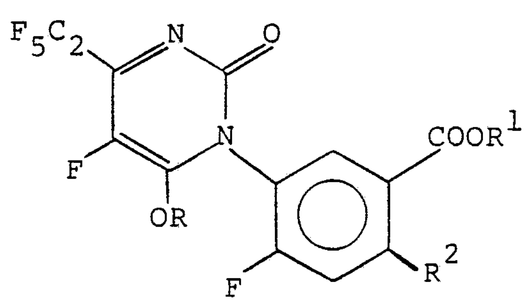

$Ib^4$

worin R, $R^1$ und $R^2$ die in der nachfolgenden Tabelle 1 angegebenen Bedeutungen besitzen:

Tabelle 1

| R | $R^1$ | $R^2$ |
|---|---|---|
| $CH_3$ | $CH_3$ | Cl |
| " | " | Br |
| " | $C_2H_5$ | Cl |
| " | " | Br |
| " | $nC_3H_7$ | Cl |
| " | " | Br |
| " | $isoC_3H_7$ | Cl |
| " | " | Br |
| " | $nC_4H_9$ | Cl |
| " | " | Br |
| " | $-CH_2OCH_3$ | Cl |
| " | " | Br |
| " | $-CH_2CH{=}CH_2$ | Cl |
| " | " | Br |
| " | $-CH_2C{\equiv}CH$ | Cl |
| " | " | Br |
| $C_2H_5$ | $CH_3$ | Cl |
| " | " | Br |
| " | $C_2H_5$ | Cl |
| " | " | Br |
| " | $isoC_3H_7$ | Cl |
| " | " | Br |
| $isoC_3H_7$ | $isoC_3H_7$ | Cl |
| " | " | Br |
| $-CH_2CH{=}CH_2$ | " | Cl |
| " | " | Br |
| $-CH_2C{\equiv}CH$ | " | Cl |
| " | " | Br |

sowie diejenigen der Formeln

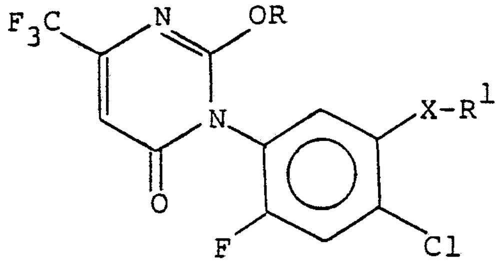

$Ia^5$

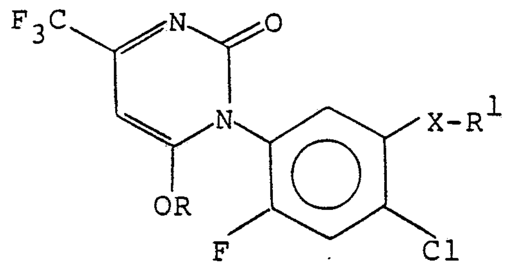

$Ib^5$

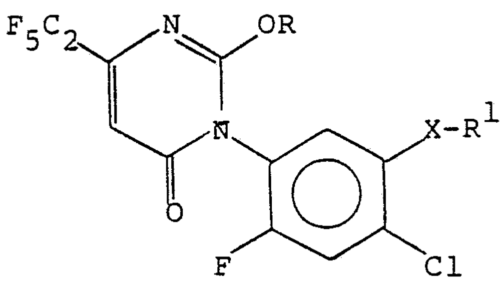

$Ia^6$

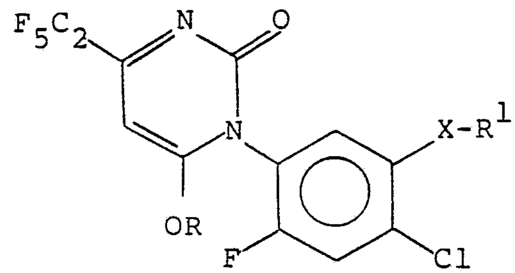

$Ib^6$

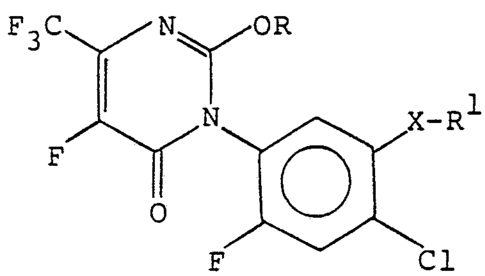

$Ia^7$

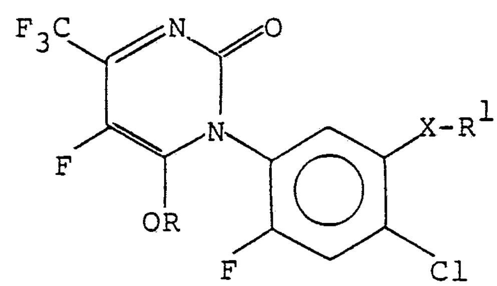

$Ib^7$

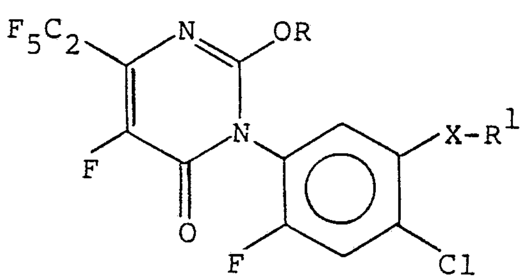

$Ia^8$

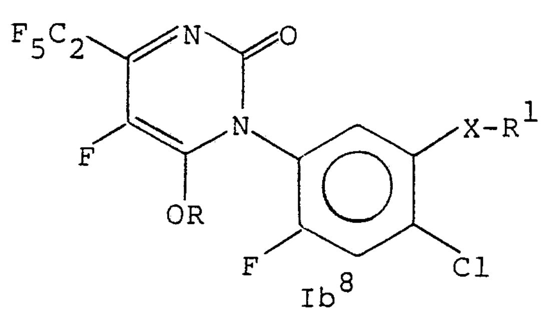

$Ib^8$

worin R, $R^1$ und X die in der folgenden Tabelle 2 angegebenen Bedeutungen besitzen:

Tabelle 2

| R | $R^1$ | X |
|---|---|---|
| $CH_3$ | $CH_3$ | O |
| " | iso$C_3H_7$ | " |
| " | $-CH_2CH{=}CH_2$ | " |
| " | $-CH_2C{\equiv}CH$ | " |
| " | $-CH_2OCH_3$ | " |
| " | $CH_3$ | O-C(O) |
| " | " | O-C(O)-O |
| $C_2H_5$ | " | O |
| " | iso$C_3H_7$ | " |
| " | $-CH_2C{\equiv}CH$ | " |
| iso$C_3H_7$ | iso$C_3H_7$ | " |
| " | $-CH_2C{\equiv}CH$ | " |
| $C_2H_5$ | $CH_3$ | O-C(O) |
| " | " | O-C(O)-O |

Das erfindungsgemässe Verfahren zur Herstellung der Enoläther ist dadurch gekennzeichnet, dass man

a) ein Uracilderivat der allgemeinen Formel

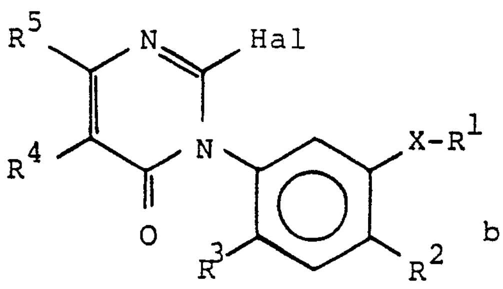

IIa

bzw.

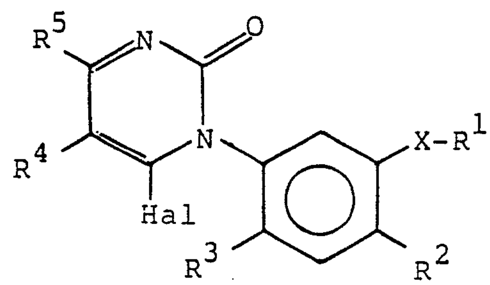

IIb

worin $R^1$-$R^5$ und X die oben angegebenen Bedeutungen besitzen und Hal Chlor oder Brom bedeutet, mit einem Alkohol ROH in Gegenwart einer organischen Base oder mit dem Metallalkoholat der allgemeinen Formel

$RO^{\ominus}M^{\oplus}$ III

worin R $C_{1-4}$-Alkyl, $C_{2-4}$-Alkenyl oder $C_{3 \text{ oder } 4}$-Alkinyl und $M^{\oplus}$ ein Aequivalent eines Metallions bedeuten,

behandelt, oder

b) ein 3-Aryluracil der oben angegebenen allgemeinen Formel I', in der X O oder C(O)-O bedeutet, unter basischen Reaktionsbedingungen einer entsprechenden Alkylierung unterwirft, oder

c) in einem Uracilderivat der allgemeinen Formel

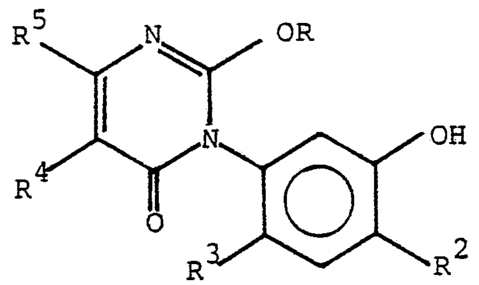

IVa

bzw.

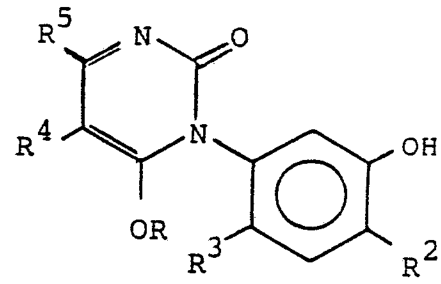

IVb

worin R, $R^2$, $R^3$, $R^4$ und $R^5$ die oben angegebenen Bedeutungen besitzen,
die Hydroxygruppe in an sich bekannter Weise durch den Substituenten X'-$R^1$ ersetzt, worin X' für O, O-C-(O) oder O-C(O)-O steht, oder

d) eine Benzoesäure der allgemeinen Formel

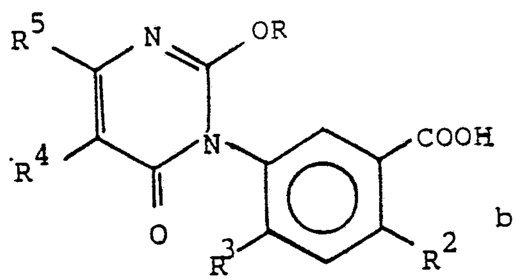

Va

bzw.

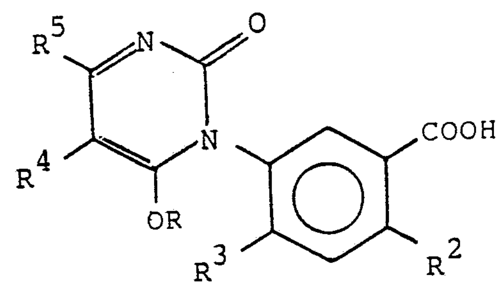

Vb

worin R, $R^2$, $R^3$, $R^4$ und $R^5$ die oben angegebenen Bedeutungen besitzen,
oder ein reaktionsfähiges Derivat davon entsprechend verestert, und dass man den gewünschten Enoläther isoliert.

Bei der Verfahrensvariante a) steht der Ausdruck "Metallion" insbesondere für ein Alkalimetallion, z.B. das Natrium-oder Kaliumion, oder ein Erdalkalimetallion, z.B. das Calcium-oder Magnesiumion. Das Natriumion ist das bevorzugte Metallion. Im Falle der Verwendung des Alkohols ROH eignet sich als Base insbesondere eine organische tertiäre Base, wie Triäthylamin oder Pyridin.

Die Umsetzung erfolgt zweckmässigerweise in einem Ueberschuss an dem entsprechenden Alkohol ROH als Verdünnungsmittel und bei Temperaturen zwischen 0°C und 50°C, vorzugsweise bei Raumtemperatur.

Die so erhaltene erfindungsgemässe Verbindung kann nach Aufarbeiten des Reaktionsgemisches nach an sich bekannten Methoden, z.B. Säulenchromatographie und/oder Kristallisieren, isoliert und gereinigt werden.

Diese Verfahrensvariante eignet sich vorzugsweise zur Herstellung derjenigen erfindungsgemässen Verbindungen Ia und Ib, in denen X O oder C(O)-O bedeutet.

Die Umsetzung nach Verfahrensvariante b) führt zu Endprodukten der Formel Ia, in denen X O oder C-(O)-O bedeutet.

Als Alkylierungsmittel (worunter Mittel zur Einführung einer Alkyl-, einer Alkenyl-bzw. einer Alkinylgruppe zu verstehen sind) für diese Verfahrensvariante wird zweckmässigerweise ein $C_{1-4}$-Alkyl-, $C_{2-4}$-Alkenyl-bzw. $C_{3\ \text{oder}\ 4}$-Alkinylhalogenid, insbesondere das diesbezügliche Chlorid oder Bromid, oder -Sulfat verwendet. Die Umsetzung wird zweckmässigerweise in Gegenwart eines inerten aprotischen oder protischen Lösungsmittels, wie eines niederen Alkohols, z.B. Aethanol; eines aliphatischen oder cyclischen Aethers, z.B. 1,2-Dimethoxyäthan, Tetrahydrofuran oder Dioxan; eines aliphatischen Ketons, z.B. Aceton; Acetonitril; Dimethylformamid; oder Dimethylsulfoxid, oder eines Gemisches eines oder mehrerer dieser

Lösungsmittel mit Wasser, sowie in Gegenwart einer Base, wie Natriumhydrid, eines Alkalimetallcarbonats, insbesondere Natriumcarbonat oder Kaliumcarbonat, oder eines Alkalimetallhydrogencarbonats, insbesondere Natriumhydrogencarbonat oder Kaliumhydrogencarbonat, bei Temperaturen zwischen 0°C und 100°C, vorzugsweise zwischen der Raumtemperatur und 50°C, durchgeführt. Aceton, Acetonitril und Dimethylformamid sind die bevorzugten Lösungsmittel. Das gewünschte Endprodukt der Formel Ia kann nach Aufarbeiten des Reaktionsgemisches von allfälligen verbleibenden Ausgangsmaterialien und/oder Nebenprodukten nach an sich bekannten Methoden, z.B. Säulenchromatographie und/oder Kristallisieren, freigesetzt werden.

Die Umsetzung nach Verfahrensvariante c) führt zu Endprodukten der Formel Ia bzw. Ib, in denen X O, O-C(O) oder O-C(O)-O bedeutet. Bei dieser Verfahrensvariante handelt es sich also je nach Natur des Restes X'-$R^1$ um eine Alkylierung (worunter die Einführung einer Alkyl-, Alkenyl-, Alkinyl-, Alkoxyalkyl-oder Alkylidenaminooxyalkylgruppe zu verstehen ist) bzw. Acylierung, die in an sich bekannter Weise durchgeführt werden kann. Nachträgliche Reaktionsstufen und/oder andersartige Substitutions-oder Additionsreaktionen an der Gruppe X'-$R^1$, die zu weiteren Gruppen X'-$R^1$ führen, sind allerdings nicht aus dieser Verfahrensvariante ausgeschlossen.

Als typisches Beispiel einer Alkylierung wird eine Verbindung der Formel IVa oder IVB in eine Verbindung der Formel Ia bzw. Ib, in der X-$R^1$ $C_{2-6}$-Alkinoxy, z.B. Propargyloxy, bedeutet, übergeführt. Dies wird zweckmässigerweise durchgeführt, indem man die Verbindung IVa oder IVb in einem inerten aprotischen, polaren Lösungsmittel, wie biespielsweise Aceton, Dimethylformamid, Dimethylsulfoxid oder Acetonitril, mit einem $C_{2-6}$ -Alkinylhalogenid, insbesondere dem Chlorid oder Bromid, und einer Base, z.B. Natrium oder Kaliumcarbonat, bei Temperaturen zwischen der Raumtemperatur und ca. 80°C behandelt.

Als typisches Beispiel einer Acylierung wird eine Verbindung der Formel IVa oder IVb in eine Verbindung der Formel Ia bzw. Ib, in der X-$R^1$ $C_{2-9}$-Alkoxycarbonyloxy, z.B. Methoxycarbonyloxy, bedeutet, übergeführt. Die Umsetzung wird zweckmässigerweise durchgeführt, indem man die Verbindung IVa oder IVb in einem inerten aprotischen Lösungsmittel, wie Dimethylformamid, Dimethylsulfoxid, Acetonitril, einem aliphatischen Aether, z.B. 1,2-Dimethoxyäthan, oder einem chlorierten, aliphatischen Kohlenwasserstoff, z.B. Methylenchlorid, mit einer Base, wie beispielsweise Natriumhydrid, Triäthylamin oder Pyridin, versetzt und anschliessend mit einem $C_{2-9}$ -Alkoxycarbonylhalogenid in demselben Lösungsmittel bei Temperaturen zwischen der Raumtemperatur und ca. 80°C behandelt. Vorzugsweise wird in Methylenchlorid in Gegenwart von Triäthylamin oder Pyridin bei Raumtemperatur gearbeitet und anschliessend Chlorameisensäuremethylester auch bei Raumtemperatur eingesetzt.

Die Umsetzung nach Verfahrensvariante d) führt zu Endprodukten der Formel Ia bzw. Ib, in denen X C-(O)-O bedeutet. Bei dieser Verfahrensvariante handelt es sich um eine Veresterung einer substituierten Benzoesäure bzw. eines reaktionsfähigen Derivats davon, die ebenfalls nach an sich bekannten Methoden durchgeführt werden kann. So wird beispielsweise ein Salz der Benzoesäure der Formel Va bzw. Vb mit einem $C_{1-8}$-Alkyl-, $C_{2-6}$-Alkenyl-, $C_{2-6}$-Alkinyl-oder $C_{2-8}$-Alkoxyalkyl-chlorid, -bromid, -jodid, -sulfat, -mesylat oder -tosylat bzw. mit dem entsprechenden Derivat des [(Alkylidenamino)oxy]methanols oder -äthanols $R^6R^7C=N-O-(CH_2)_m-OH$ in einem inerten Verdünnungsmittel bei Temperaturen zwischen der Raumtemperatur und 100°C, z.B. bei der Rückflusstemperatur des Reaktionsgemisches, vorzugsweise im Temperaturbereich von 40°C bis 70°C, umgesetzt. Es kommen als Salze der Benzoesäure der Formel Va bzw. Vb insbesondere Alkalimetallsalze, z.B. das Natrium-, Kalium-oder Lithiumsalz, Erdalkalimetallsalze, z.B. das Magnesium-, Calcium-oder Bariumsalz, und Salze mit organischen Basen, wie tertiäre Amine, z.B. Triäthylamin, 1,5-Diaza-bicyclo[4,3,0]non-5-en, 1,8-Diaza-bicyclo[5,4,0]undec-7-en und 1,4-Diaza-bicyclo-[2,2,2]octan, in Frage, wobei die Alkalimetallsalze, insbesondere das Natriumsalz und das Kaliumsalz, bevorzugt sind. Die verwendbaren Verdünnungsmittel sind vorzugsweise inerte organische Lösungsmittel, wie niedere Alkanole, z.B. Aethanol, aliphatische und cyclische Aether, z.B. Diäthyläther, Tetrahydrofuran und Dioxan, Ketone, z.B. Aceton und 2-Butanon, Dimethylformamid, Dimethylsulfoxid und Hexamethylphosphorsäuretriamid. Das Salz kann in situ hergestellt werden, indem man die Säure mit einer geeigneten anorganischen Base, z.B. einem Alkalimetall-oder Erdalkalimetallcarbonat oder -hydrogencarbonat, bzw. organischen Base, zum Salz umsetzt und dies anschliessend im gleichen Reaktionsmedium mit dem zweiten Reaktionspartner reagieren lässt.

Im Falle der Verwendung eines Säurehalogenids der Benzoesäure der Formel Va bzw. Vb als reaktionsfähiges Derivat wird dies zweckmässigerweise mit einem $C_{1-8}$ -Alkanol, $C_{2-6}$-Alkenol, $C_{2-6}$-Alkinol, C $_{2-8}$-Alkoxyalkanol oder Alkohol $R^6R^7C=N-O-(CH_2)_m-OH$ in einem inerten organischen Lösungsmittel, wie einem aliphatischen oder cyclischen Aether, z.B. Diäthyläther, Tetrahydrofuran oder Dioxan, einem aliphatischen oder aromatischen Kohlenwasserstoff, z.B. n-Hexan, Benzol oder Toluol, oder einem halogenierten, insbesondere chlorierten, Kohlenwasserstoff, z.B. Methylenchlorid, Chloroform oder Tetrachlorkohlenstoff,

bei Temperaturen von etwa -20°C bis 100°C, vorzugsweise von 0°C bis 50°C, umgesetzt. Zudem wird zweckmässigerweise in Gegenwart eines säurebindenden Mittels gearbeitet, wie einer organischen Base, z.B. Triäthylamin, Pyridin, 1,5-Diaza-bicyclo[4,3,0]non-5-en, 1,8-Diaza-bicyclo[5,4,0]undec-7-en oder 1,4-Diaza-bicyclo[2,2,2]octan. Das Säurehalogenid ist vorzugsweise das Säurechlorid.

Als weitere in Frage kommende reaktionsfähige Derivate der Benzoesäure der Formel Va bzw. Vb seien der entsprechende O-Acyl-1,3-dicyclohexylisoharnstoff und das entsprechende N-Acylimidazol oder Säureanhydrid genannt. Solche Derivate können wie das Säurehalogenid mit einem $C_{1-8}$-Alkanol, $C_{2-6}$-Alkenol, $C_{2-6}$-Alkinol, $C_{2-8}$-Alkoxyalkanol oder Alkohol $R^6R^7C=N-O-(CH_2)_m-OH$ umgesetzt werden, um zu den gewünschten Benzoesäureestern zu gelangen. In diesen Fällen erübrigt sich jedoch die Verwendung eines säurebindenden Mittels.

Die Ausgangsmaterialien der Formeln IIa und IIb, die neu sind, können aus den oben angegebenen 3-Aryluracilen der Formel I' hergestellt werden, und zwar dadurch, dass man ein solches 3-Aryluracil mit einem Chlorierungs-bzw. Bromierungsmittel behandelt. Zu diesem Zwecke wird insbesondere Phosphorpentachlorid oder Phosphoroxychlorid bzw. Phosphorpentabromid oder Phosphorylbromid als Halogenierungsmittel verwendet. Gegebenenfalls wird ein Gemisch von Phosphorpentachlorid und Phosphoroxychlorid bzw. von Phosphorpentabromid und Phosphorylbromid eingesetzt, wobei ein Ueberschuss an Phosphoroxychlorid bzw. Phosphorylbromid als Verdünnungsmittel dienen kann. Die Chlorierung bzw. Bromierung kann in Gegenwart eines inerten Verdünnungsmittels, insbesondere eines aprotischen organischen Lösungsmittels, wie eines aliphatischen oder aromatischen Kohlenwasserstoffes, z.B. n-Hexan, Benzol, Toluol oder eines Xylols; eines halogenierten aliphatischen Kohlenwasserstoffes, z.B. Methylenchlorid, Chloroform oder 1,2-Dichloräthan; oder eines halogenierten aromatischen Kohlenwasserstoffes, z.B. Chlorbenzol, sowie - insbesondere im Falle von Phosphoroxychlorid oder Phosphorylbromid - in Gegenwart einer organischen Base, wie eines tertiären Amins, z.B. Pyridin oder N,N-Dimethylanilin, durchgeführt werden. Die Reaktionstemperaturen liegen im allgemeinen zwischen 0°C und der Rückflusstemperatur des Reaktionsgemisches, vorzugsweise zwischen 20°C und 70°C.

Auf diese Weise wird in gewissen Fällen ein Gemisch der beiden Verbindungen IIa und IIb erhalten. Solche Gemische können gegebenenfalls aufgetrennt und die einzelnen Isomeren der Umsetzung mit der Verbindung III unterworfen werden, oder aber man kann, vorzugsweise, das Isomerengemisch IIa/IIb mit der Verbindung III umsetzen und danach das Endprodukt gegebenenfalls in die einzelnen Verbindungen Ia und Ib auftrennen. Solche Trennungen können nach an sich bekannten Methoden durchgeführt werden.

Die neuen Verbindungen IIa und IIb bilden einen weiteren Gegenstand der vorliegenden Erfindung.

Die 3-Aryluracile der Formel I' sind ebenfalls neu; diese können in an sich bekannter Weise hergestellt werden, beispielsweise gemäss dem nachfolgenden Reaktionsschema, in dem $R^1$-$R^5$ und X die oben angegebenen Bedeutungen besitzen, und $R^8$ und $R^9$ jeweils nieder Alkyl, vorzugsweise $C_{1-4}$-Alkyl, bedeuten:

Reaktionsschema

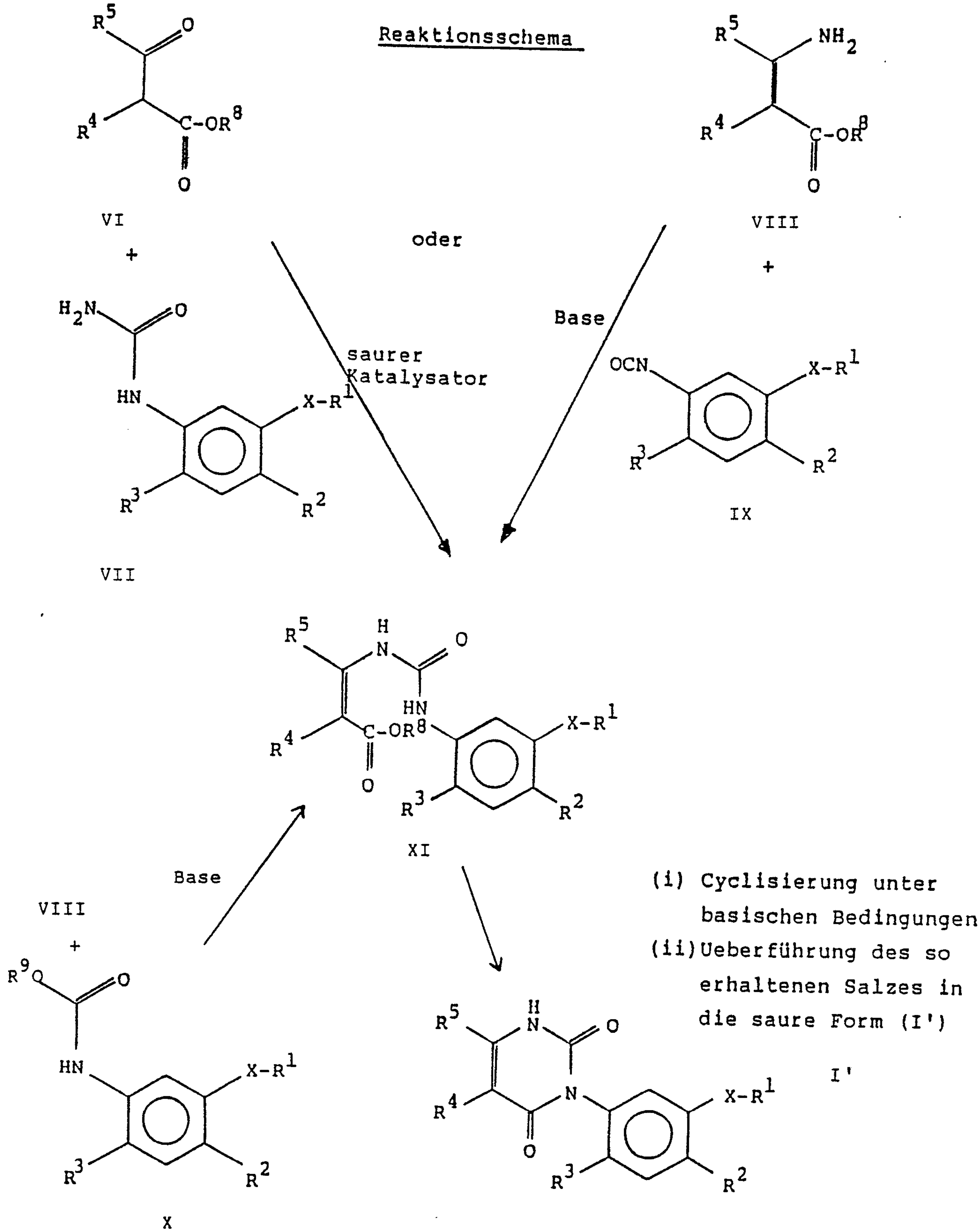


VI + VII

VIII + IX

VIII + X

XI

I'

Die Verbindungen der Formeln VI und VII werden zweckmässigerweise in einem im wesentlichen wasserfreien Verdünnungsmittel und in Gegenwart eines sauren Katalysators bei erhöhter Temperatur miteinander umgesetzt. Als Verdünnungsmittel kommen insbesondere mit Wasser Azeotrope bildende organische Lösungsmittel, wie Aromaten, z.B. Benzol, Toluol und Xylole; halogenierte Kohlenwasserstoffe, wie Methylenchlorid, Chloroform, Tetrachlorkohlenstoff und Chlorbenzol; aliphatische und cyclische Aether, wie 1,2-Dimethoxyäthan, Tetrahydrofuran und Dioxan; und Cyclohexan, und als saure Katalysatoren insbesondere starke Mineralsäuren, wie Schwefelsäure und Salzsäure; organische Säuren, wie p-Toluolsulfonsäure; Phosphor enthaltende Säuren, wie Orthophosphorsäure und Polyphosphorsäure; und saure Kationenaustauscher, wie "Amberlyst 15" (Fluka), in Frage. Man arbeitet im allgemeinen in einem Temperaturbereich von etwa 70°C bis 120°C, vorzugsweise bei der Rückflusstemperatur der Reaktionsgemisches. Unter diesen Reaktionsbedingungen wird die erwünschte rasche Entfernung des in der Reaktion gebildeten Wassers erzielt.

Die Umsetzung der Verbindungen der Formeln VIII und IX miteinander erfolgt zweckmässigerweise in Gegenwart eines im wesentlichen wasserfreien aprotischen organischen Lösungsmittels, wie eines aliphatischen oder cyclischen Aethers, z.B. Diäthyläther, 1,2-Dimethoxyäthan, Tetrahydrofuran oder Dioxan, eines aliphatischen oder aromatischen Kohlenwasserstoffs, z.B. n-Hexan, Benzol, Toluol oder eines Xylols, oder eines halogenierten, aliphatischen Kohlenwasserstoffs, z.B. Methylenchlorid, Chloroform, Tetrachlorkohlenstoff oder 1,2-Dichloräthan; eines aprotischen, polaren Lösungsmittels, wie Dimethylformamid, Hexamethylphosphorsäuretriamid oder Dimethylsulfoxid; oder eines Gemisches zweier oder mehrerer der erwähnten Lösungsmittel, sowie gegebenenfalls in Gegenwart einer Base, insbesondere einer organischen tertiären Base, wie Triäthylamin oder Pyridin, wobei letzteres sowohl als Lösungsmittel wie auch als Base dienen kann, oder eines Metallhydrids, wie Natrium-oder Kaliumhydrid. Die Reaktionstemperaturen sind vorzugsweise im Bereich von etwa -70°C bis 50°C, wobei besonders bevorzugt bei Temperaturen zwischen -30°C und der Raumtemperatur gearbeitet wird.

Die Umsetzung der Verbindungen der Formeln VIII und X miteinander erfolgt zweckmässigerweise in einem aprotischen polaren Verdünnungsmittel, wie Dimethylformamid, 2-Butanon, Dimethylsulfoxid oder Acetonitril, in Gegenwart einer Base, wie eines Alkalimetall-oder Erdalkalimetallalkoholats oder -carbonats, insbesondere Natriumalkanolat oder -carbonat, oder eines Metallhydrids, insbesondere Lithium oder Natriumhydrid, bei Temperaturen zwischen 80°C und 180°C, vorzugsweise bei der Rückflusstemperatur des Reaktionsgemisches. Falls ein Alkoholat als Base verwendet wird, destilliert man zweckmässigerweise den im Laufe der Reaktion freigesetzten Alkohol kontinuierlich ab. Bei dieser Methode wird die so hergestellte Verbindung der Formel XI in der Regel in situ hergestellt, da die verwendeten Reaktionsbedingungen die Cyclisierung der Verbindung XI über das Salz zu der Verbindung der Formel I' begünstigen.

Die Cyclisierung der Verbindung der Formel XI, wenn diese nicht bereits in situ geschieht, kann zweckmässigerweise durchgeführt werden, indem man diese in einem inerten protischen organischen Lösungsmittel, wie einem Alkohol, z.B. Methanol, Aethanol oder Isopropanol; einem inerten aprotischen organischen Lösungsmittel, wie einem aliphatischen oder cyclischen Aether, z.B. 1,2-Dimethoxyäthan, Tetrahydrofuran oder Dioxan, oder einem Aromaten, z.B. Benzol oder Toluol; einem inerten aprotischen, polaren organischen Lösungsmittel, z.B. Dimethylformamid oder Dimethylsulfoxid, wobei solche Lösungsmittel gegebenenfalls im Zweiphasen-Gemisch mit einem Kohlenwasserstoff, z.B. n-Hexan, verwendet werden können; oder Wasser mit einer Base bei Temperaturen zwischen Raumtemperatur und der Rückflusstemperatur des Reaktionsgemisches behandelt. Als Basen kommen vorzugsweise Natriumalkoholate, Alkalimetallhydroxide, insbesondere Natriumhydroxid und Kaliumhydroxid, Alkalimetallcarbonate, insbesondere Natriumcarbonat und Kaliumcarbonat, und Natriumhydrid in Betracht. Bei der Verwendung der letztgenannten Base ist das Lösungsmittel vorzugsweise ein aliphatischer oder cyclischer Aether, Dimethylformamid oder Dimethylsulfoxid.

Nach Beendigung der Cyclisierung liegt das Produkt je nach Natur der verwendeten Base in Form des entsprechenden Metallsalzes vor, beispielsweise im Falle der oben erwähnten Basen in Form des entsprechenden Alkalimetallsalzes. Das nach der Cyclisierung entstandene Gemisch wird dann angesäuert, um die Ueberführung des Salzes in die Verbindung I' zu erzielen. Zu diesem Zwecke verwendet man vorzugsweise eine Mineralsäure, wie Salzsäure, oder eine starke organische Säure, wie Essigsäure oder p-Toluolsulfonsäure. Das Produkt kann dann in an sich bekannter Weise isoliert und gegebenenfalls gereinigt werden.

Auch die Uracilderivate der Formeln IVa und IVb sind neu; diese können durch säurekatalysierte Hydrolyse der entsprechenden geschützten Phenole der Formel

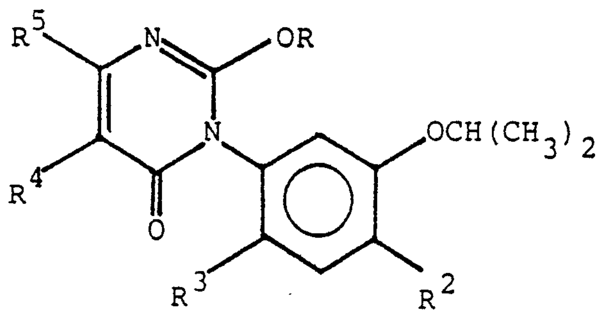

bzw.

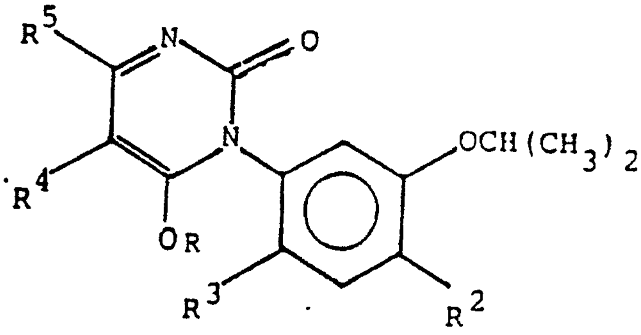

Ia'　　　Ib'

worin R, $R^2$, $R^3$, $R^4$ und $R^5$ die oben angegebenen Bedeutungen besitzen, hergestellt werden. Die Hydrolyse erfolgt zweckmässigerweise in Gegenwart von Schwefelsäure als saurem Katalysator, in einem chlorierten aliphatischen Kohlenwasserstoff, vorzugs weise Methylenchlorid, als Lösungsmittel und bei Temperaturen zwischen -30°C und 30°C, vorzugsweise zwischen 0°C und Raumtemperatur. Ohne zusätzliches Lösungsmittel kann überschüssige Schwefelsäure selbst als Lösungsmittel dienen. Bei den obigen Ausgangsmaterialien der Formeln Ia' und Ib' handelt es sich um eine Untergruppe von Verbindungen der Formel Ia bzw. Ib. Die neuen Verbindungen IVa und IVb bilden einen weiteren Gegenstand der vorliegenden Erfindung.

Ebenfalls neu sind die Benzoesäuren der Formeln Va und Vb sowie deren reaktionsfähige Derivate. Die Benzoesäuren können durch Hydrolyse der entsprechenden Enoläther der oben angegebenen Formeln Ia und Ib, in denen X C(O)-O bedeutet, hergestellt werden. Diese Hydrolyse kann nach an sich bekannten Methoden erfolgen, insbesondere unter Verwendung einer anorganischen Säure und gegebenenfalls in Gegenwart eines organischen Lösungsmittels. Als Säuren kommen vorzugsweise Schwefelsäure, als organische Lösungsmittel gegebenenfalls chlorierte aliphatische Kohlenwasserstoffe, z.B. Methylenchlorid und Tetrachlormethan, in Frage. Ohne zusätzliches Lösungsmittel kann überschüssige Schwefelsäure selbst als Lösungsmittel dienen. Die Reaktionstemperaturen liegen im allgemeinen zwischen -30°C und 30°C, vorzugsweise zwischen 0°C und der Raumtemperatur. Ausgehend von den Benzoesäuren können deren reaktionsfähige Derivate nach an sich bekannten Methoden hergestellt werden.

Die in den Verfahrensvarianten a) und b) benötigten Alkohole ROH, Alkoholate der Formel III bzw. Alkylierungsmittel sowie die in dem Reaktionsschema involvierten Ausgangsmaterialien der Formeln VI, VII, VIII, IX und X sind entweder bekannt oder können nach an sich bekannten Methoden hergestellt werden.

Die erfindungsgemässen Enoläther besitzen herbizide Eigenschaften und eignen sich zur Bekämpfung von Unkräutern, einschliesslich Ungräsern, insbesondere Setaria faberii, Digitaria sanguinalis, Poa annua, Chenopodium album, Amaranthus retroflexus, Abutilon theopharasti, Sinapsis alba, Datura stramonium und Sorghum halepense, in diversen Nutzpflanzenkulturen, insbesondere in Baumwolle-, Reis-, Mais-, Getreide- und Soyakulturen. Zudem sind die Verbindungen sowohl Vorauflauf-als auch Nachauflauf-Herbizide.

Auch die Verbindungen der Formeln IVa und IVb besitzen herbizide Eigenschaften und können auf ähnliche Weise wie die erfindungsgemässen Enoläther zur Bekämpfung von Unkräutern, insbesondere den obenerwähnten, eingesetzt werden. Im folgenden werden die erfindungsgemässen Enoläther (Verbindungen der Formeln Ia und Ib) und Verbindungen der Formeln IVa und IVb lediglich als (erfindungsgemässe) Wirkstoffe bezeichnet.

Ueblicherweise genügt eine Konzentration von 0,005-6,0 kg Wirkstoff/ha, vorzugsweise 0,05-2,0 kg Wirkstoff/ha, um den gewünschten herbiziden Effekt zu erzielen.

Das erfindungsgemässe Unkrautbekämpfungsmittel ist dadurch gekennzeichnet, dass es eine wirksame Menge mindestens eines erfindungsgemässen Wirkstoffes sowie Formulierungshilfsstoffe enthält. Das Mittel enthält zweckmässigerweise zumindest einen der folgenden Formulierungshilfsstoffe: feste Trägerstoffe; Lösungs-bzw. Dispersionsmittel; Tenside (Netz-und Emulgiermittel); Dispergatoren (ohne Tensidwirkung); und Stabilisatoren. Unter Verwendung solcher und anderer Hilfsstoffe können die erfindungsgemässen Wirkstoffe in die üblichen Formulierungen übergeführt werden, wie Stäube, Pulver, Granulate, Lösungen, Emulsionen, Suspensionen, emulgierbare Konzentrate, Pasten und dergleichen.

Die erfindungsgemässen Wirkstoffe sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs-bzw. Dispersionsmitteln, eventuell unter Verwendung von Tensiden als Netz-oder Emulgiermitteln und/oder von Dispergatoren, durch Verdünnen bereits vorbereiteter emulgierbarer Konzentrate mit Lösungs-bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorrillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs-bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs-bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkte von mindestens 50°C aufweisen, und Wasser. Unter den Lösungs-bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasser stoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz-und Emulgiermittel) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium-und Ammoniumsalze von Ligninsulfonsäuren, Natriumsalze von Maleinsäureanhydrid-Diisobutylen-Copolymeren, Natrium-und Ammoniumslaze von sulfonierten Polykondensationsprodukten aus Naphthalin und Formaldehyd, und Sulfitablaugen.

Als Dispergatoren, die sich insbesondere als Verdickungs-bzw. Antiabsetzmittel eignen, können z.B. Methyl cellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylonitrilsäureester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den erfindungsgemässen Wirkstoffen Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung der Wirkungsspektrums.

Die erfindungsgemässen Unkrautbekämpfungsmittel enthalten im allgemeinen zwischen 0,01 und 95 Gewichtsprozent, vorzugsweise zwischen 0,5 und 75 Gewichtsprozent eines bzw. mehrerer erfindungsgemässen Wirkstoffe. Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 1 und 50 Gewichtsprozent, insbesondere zwischen 10

und 20 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,01 bis 10 Gewichtsprozent, insbesondere ca. 0,5 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der erfindungsgemässe Wirkstoff mit festem Trägerstoff vermischt werden, z.B. durch Zusammenmahlen; oder man kann den festen Trägerstoff mit einer Lösung oder Suspension des Wirkstoffes imprägnieren und dann das Lösungs-bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Der erfindungsgemässe Wirkstoff kann auch mit einem Tensid und einem festen Trägerstoff zur Bildung eines netzbaren Pulvers vermischt werden, welches in Wasser dispergierbar ist, oder er kann mit einem festen vorgranulierten Trägerstoff zur Bildung eines granulatförmigen Produktes vermischt werden.

Wenn gewünscht, kann der erfindungsgemässe Wirkstoff in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer wirksamen Menge eines erfindungsgemässen Wirkstoffes bzw. eines erfindungsgemässen Unkrautbekämpfungsmittels behandelt.

Die nachfolgenden Beispiele dienen zur näheren Erläuterung der Erfindung.

I. Herstellung der erfindungsgemässen Enoläther:

Beispiel 1

Zu einer Lösung von 3,35 g 2-Chlor-5-3,6-dihydro-2,6-dioxo-4-pentafluoräthyl -1(2H)-pyrimidinyl]-4-fluorbenzoesäure-isopropylester und 10 ml Phosphoroxychlorid werden unter Rühren bei 25°C 1,3 ml Dimethylanilin zugefügt, und das Reaktionsgemisch wird 5 Stunden bei 75°C gerührt. Anschliessend werden weitere 1,3 g Dimethylanilin zugefügt, und das Gemisch wird 5 Stunden bei 75°C gerührt und danach 1 Stunde bei der Rückflusstemperatur erhitzt. Dann wird das Gemisch unter vermindertem Druck bei 70°C zur Trockene eingedampft und der Rückstand in 100 ml Diäthyläther gelöst und die Lösung dreimal mit je 50 ml Eiswasser ausgeschüttelt. Die organische Phase wird über wasserfreiem Natriumsulfat getrocknet und unter vermindertem Druck zur Trockene eingedampft.

Der Rückstand, ein Gemisch von 2-Chlor-5-[2-chlor-6-oxo-4-pentafluoräthyl -1(6H)-pyrimidinyl]-4-fluorbenzoesäure-isopropylester und 2-Chlor-5-[6-chloro-2-oxo-4-pentafluoräthyl -1(2H)-pyrimidinyl]-4-fluorbenzoesäure-isopropylester, wird in 20 ml Methanol gelöst, und der Lösung werden unter Rühren bei 25°C 3,8 ml einer 2N-Natriummethylat-Lösung zugefügt. Die Temperatur des Reaktionsgemisches steigt auf 30°C an. Anschliessend wird 30 Minuten nachgerührt und unter vermindertem Druck zur Trockene eingedampft. Der Rückstand wird an einer Kieselgel-Säule unter Verwendung von n-Hexan/Diäthyläther (3:1) chromatographisch aufgetrennt. Die erste Fraktion, die noch etwas Diäthylanilin enthält, wird in Diäthyläther gelöst, und die Lösung mit 2N-Salzsäure ausgeschüttelt, mit Wasser neutral gewaschen und über wasserfreiem Natriumsulfat getrocknet. Die organische Phase wird unter vermindertem Druck zur Trockene eingedampft und der Rückstand aus n-Hexan umkristallisiert. Man erhält den 2-Chlor-4-fluor-5-[2-methoxy-6-oxo-4-pentafluoräthyl -1(6H)-pyrimidinyl]-benzoesäure-isopropylester, Smp. 82-84°C. Durch weiteres Eluieren der Säule mit n-Hexan/Diäthyläther (1:2) erhält man die zweite Fraktion, welche aus Diäthyläther/n-Hexan umkristallisiert wird. Auf diese Weise erhält man auch noch den 2-Chlor-4-fluor-5-[6-methoxy-2-oxo-4-pentafluoräthyl -1(2H)-pyrimidinyl]-benzoesäure-isopropylester, Smp. 127-129°C.

Beispiel 2

Zu einer Lösung von 0,21 g einer 55%-igen Natriumhydrid-Dispersion in 20 ml Isopropanol werden unter Rühren und Kühlen bei 0°C 2,0 g 2-Chlor-5-[2-chlor-6-oxo-4-trifluormethyl-1(6H)-pyrimidinyl] -4-fluorbenzoesäure-isopropylester zugefügt. Das Reaktionsgemisch wird 10 Minuten bei Raumtemperatur nachgerührt und unter vermindertem Druck zur Trockene eingedampft. Man löst den Rückstand in 50 ml Diäthyläther und wäscht die Lösung zweimal mit je 25 ml Wasser. Die organische Phase wird über wasserfreiem Natriumsulfat getrocknet und zur Trockene eingedampft und der Rückstand an einer Kieselgel-Säule unter Verwendung von n-Hexan/Diäthyläther (3:1) als Laufmittel chromatographisch gereinigt. Man erhält den 2-Chlor-4-fluor-5-[2-isopropoxy-6-oxo-4-trifluormethyl -1(6H)-pyrimidinyl]-benzoesäure-isopropyl ester, $^1$H-NMR ($CDCl_3$, 400 MHz): 7,81 ppm (d,1H), 7.38 ppm (d,1H), 6.58 ppm (s,1H), 5.40 ppm (m,1H), 5,26 ppm (m,1H), 1.38 ppm (d,3H), 1.37 ppm (d,3H), 1,29 ppm (d,3H), 1,28 ppm (d,3H).

In analoger Weiser erhält man beim Einsatz von:

-2-Chlor-5-[2-chlor-6-oxo-4-trifluormethyl-1(6H)-pyrimidinyl]-4-fluorbenzoesäure-isopropylester und Aethanol den 5-[2-Aethoxy-6-oxo-4-trifluormethyl-1(6H)-pyrimidinyl]-2-chlor-4-fluorbenzoesäure-isopropylester, Smp. 115-117°C;

-2-Chlor-5-[2-chlor-6-oxo-4-trifluormethyl-1(6H)-pyrimidinyl]-4-fluorbenzoesäure-isopropylester und einer Lösung von Natriumhydrid in Allylalkohol den 5-[2-Allyloxy-6-oxo-4-trifluormethyl-1(6H)-pyrimidinyl]-2-chlor-4-fluorbenzoesäure-isopropylester, Smp. 94-96°C;

-2-Chlor-5-[2-chlor-6-oxo-4-trifluormethyl-1(6H)-pyrimidinyl]-4-fluorbenzoesäure-isopropylester und Propargylalkohol mit Pyridin den 2-Chlor-4-fluor-5-[6-oxo-2-(2-propinyloxy)-4-trifluormethyl-1(6H)-pyrimidinyl]-benzoesäure-isopropylester, Smp. 117°-119°C;

-2-Chlor-5-[2-chlor-6-oxo-4-trifluormethyl-1(6H)-pyrimidinyl]-4-fluorbenzoesäure-äthylester und Natriumäthylat in Aethanol den 5-[2-Aethoxy-6-oxo-4-trifluormethyl-1(6H)-pyrimidinyl]-2-chlor-4-fluorbenzoesäure-äthylester, Smp. 94-96°C;

-2-Chlor-5-[2-chlor-6-oxo-4-pentafluoräthyl-1(6H)-pyrimidinyl]-4-fluorbenzoesäure-isopropylester und Propargylalkohol mit Pyridin den 2-Chlor-4-fluor-5-[6-oxo-4-pentafluoräthyl-2-(2-propinyloxy)-1(6H)-pyrimidinyl]-benzoesäure-isopropylester, $^1$H-NMR ($CDCl_3$, 400 MHz): 7,85 ppm (d,1H), 7,41 ppm (d,1H), 6,71 ppm (s,1H), 5,27 ppm (m,1H), 5,00 ppm (d,2H), 2.53 ppm (t,1H), 1,39 ppm (d,3H), 1,38 ppm (d,3H);

-2-Chlor-5-[2-chlor-6-oxo-4-pentafluoräthyl-1(6H)-pyrimidinyl]-4-fluorbenzoesäure-isopropylester und Natriumäthylat in Aethanol den 5-[2-Aethoxy-6-oxo-4-pentafluoräthyl-1(6H)-pyrimidinyl]-2-chlor-4-fluorbenzoesäure-isopropylester, $^1$H-NMR ($CDCl_3$, 400 MHz): 7,83 ppm (d,1H), 7,39 ppm (d,1H), 6,65 ppm (s,1H), 5,72 ppm (m,1H), 4,48 ppm (m,2H), 1,38 ppm (d,3H), 1,37 ppm (d,3H), 1,29 ppm (t,3H);

-2-Chlor-5-[2-chlor-6-oxo-4-pentafluoräthyl-1(6H)-pyrimidinyl]-4-fluorbenzoesäure-isopropylester und Natriumisopropylat in Isopropanol den 2-Chlor-4-fluor-5-[2-isopropoxy-6-oxo-4-pentafluoräthyl-1(6H)-pyrimidinyl]-benzoesäure-isopropylester, $^1$H-NMR ($CDCl_3$, 400 MHz): 7,83 ppm (d,1H), 7,39 ppm (d,1H), 6,64 ppm (s,1H), 5,34 ppm (m,1H), 5,27 ppm (m,1H), 1,39 ppm (d,3H), 1,38 ppm (d,3H), 1,28 ppm (d,3H), 1,27 ppm (d,3H);

-2-Chlor-5-[2-chlor-6-oxo-4-pentafluoräthyl-1(6H)-pyrimidinyl]-4-fluorbenzoesäure-isopropylester und einer Lösung von Natriumhydrid in Allylalkohol den 5-[2-Allyloxy-6-oxo-4-pentafluoräthyl-1(6H)-pyrimidinyl]-2-chlor-4-fluorbenzoesäure-isopropylester, $^1$H-NMR ($CDCl_3$, 400 MHz): 7,84 ppm (d,1H), 7,40 ppm (d,1H), 6,67 ppm (s,1H), 5,87 ppm (m,1H), 5,21-5,32 ppm (m,3H), 4,89 ppm (m,2H), 1,38 ppm (d,3H), 1,37 ppm (d,3H);

-2-Chlor-1-(4-chlor-2-fluor-5-isopropoxyphenyl)-4-pentafluoräthyl-6(1H)-pyrimidinon und Natriumäthylat in Aethanol das 2-Aethoxy-1-(4-chlor-2-fluor-5-isopropoxyphenyl)-4-pentafluoräthyl-6(1H)-pyrimidinon, $^1$H-NMR ($CDCl_3$, 400 MHz): 7,31 ppm (d,1H), 6,81 ppm (d,1H), 6,64 ppm (s,1H), 4,39-4,55 ppm (m,3H), 1,39 ppm (d,3H), 1,38 ppm (d,3H), 1,29 ppm (t,3H);

-2-Chlor-1-(4-chlor-2-fluor-5-isopropoxyphenyl)-4-pentafluoräthyl-6(1H)-pyrimidinon und Natriumisopropylat in Iso propanol das 1-(4-Chlor-2-fluor-5-isopropoxyphenyl)-2-isopropoxy-4-pentafluoräthyl-6(1H)-pyrimidinon, $^1$H-NMR ($CDCl_3$, 400 MHz): 7,30 ppm (d,1H), 6,81 ppm (d,1H), 6,62 ppm (s,1H), 5,33 ppm (m,1H), 4,47 ppm (m,1H), 1,38 ppm (d,3H), 1,37 ppm (d,3H), 1,28 ppm (d,3H), 1,27 ppm (d,3H);

-2-Chlor-5-[2-chlor-6-oxo-4-trifluormethyl-1(6H)-pyrimidinyl]-4-fluorbenzoesäure-methylester und Natriumäthylat in Aethanol den 5-[2-Aethoxy-6-oxo-4-trifluormethyl-1(6H)-pyrimidinyl]-2-chlor-4-fluorbenzoesäure-methylester, Smp. 96-98°C;

-2-Chlor-1-(4-chlor-2-fluor-5-isopropoxyphenyl)-4-pentafluoräthyl-6(1H)-pyrimidinon und Natriumpropylat in n-Propanol das 1-(4-Chlor-2-fluor-5-isopropoxyphenyl)-4-pentafluoräthyl-2-(n-propoxy)-6(1H)-pyrimidinon, $^1$H-NMR ($CDCl_3$, 400 MHz): 7,31 ppm (d,1H), 6,82 ppm (d,1H), 6,63 ppm (s,1H), 4,46 ppm (m,1H), 4,36 ppm (m,2H), 1,66 ppm (m,2H), 1,38 ppm (d,3H), 1,37 ppm (d,3H), 0,85 ppm (t,3H);

-2-Chlor-1-(4-chlor-2-fluor-5-isopropoxyphenyl)-4-trifluormethyl-6(1H)-pyrimidinon und Natriumäthylat in

Aethanol das 2-Aethoxy-1-(4-chlor-2-fluor-5-isopropoxyphenyl)-4-trifluormethyl-6(1H)-pyrimidinon, Smp. 101-103°C;

-2-Chlor-5-[2-chlor-5-fluor-6-oxo-4-trifluormethyl-1(6H)-pyrimidinyl]-4-fluorbenzoesäure-isopropylester und Natriumäthylat in Aethanol den 5-[2-Aethoxy-5-fluor-6-oxo-4-trifluormethyl-1(6H)-pyrimidinyl]-2-chlor-4-fluor-benzoesäure-isopropylester, $^1$H-NMR ($CDCl_3$, 400 MHz): 7,83 ppm (d,1H), 7,40 ppm (d,1H), 5,27 ppm (m,1H), 4,48 ppm (m,2H), 1,39 ppm (d,3H), 1,38 ppm (d,3H), 1,29 ppm (t,3H);

-2-Chlor-1-(4-chlor-2-fluor-5-isopropoxyphenyl)-4-trifluormethyl-6(1H)-pyrimidinon und Natriumpropylat in n-Propanol das 1-(4-Chlor-2-fluor-5-isopropoxyphenyl)-2-propoxy-4-trifluormethyl-6(1H)-pyrimidinon, $^1$H-NMR ($CDCl_3$, 400 MHz): 7,31 ppm (d,1H), 6,80 ppm (d,1H), 6,59 ppm (s,1H), 4,31-5,51 ppm (m,3H), 1,67 ppm (m,2H), 1,38 ppm (d,3H), 1,37 ppm (d,3H), 0,85 ppm (t,3H);

-2-Chlor-5-[2-chlor-5-fluor-6-oxo-4-trifluormethyl-1(6H)-pyrimidinyl]-4-fluorbenzoesäure-isopropylester und eine Lösung von Natriumhydrid in Allylalkohol den 5-[2-Allyloxy-5-fluor-6-oxo-4-trifluormethyl-1(6H)-pyrimidinyl]-2-chlor-4-fluorbenzoesäure-isopropylester, $^1$H-NMR ($CDCl_3$, 400 MHz): 7,84 ppm (d,1H), 7,41 ppm (d,1H), 5,87 ppm (m,1H), 5,24-5,32 ppm (m,3H), 4,89 ppm (m,2H), 1,38 ppm (d,3H), 1,39 ppm (d,3H);

-2-Chlor-5-[2-chlor-5-fluor-6-oxo-4-trifluormethyl-1(6H)-pyrimidinyl]-4-fluorbenzoesäure-isopropylester und Propargylalkohol mit Pyridin den 2-Chlor-4-fluor-5-[5-fluor-6-oxo-2-(2-propinyloxy)-4-trifluormethyl-1(6H)-pyrimidinyl]-benzoesäure-isopropylester, $^1$H-NMR ($CDCl_3$, 400 MHz): 7,85 ppm (d,1H), 7,43 ppm (d,1H), 5,27 ppm (m,1H), 5,01 ppm (d,2H), 2,54 ppm (t,1H), 1,39 ppm (d,3H), 1,38 ppm (d,3H);

-1-(4-Brom-2-fluor-5-isopropoxyphenyl)-2-chlor-4-trifluormethyl-6(1H)-pyrimidinon und Natriummethylat in Methanol das 1-(4-Brom-2-fluor-5-isopropoxyphenyl)-2-methoxy-4-trifluormethyl-6(1H)-pyrimidinon, Smp. 137°C;

-1-(4-Brom-2-fluor-5-isopropoxyphenyl)-2-chlor-4-trifluormethyl-6(1H)-pyrimidinon und Natriumäthylat in Aethanol das 2-Aethoxy-1-(4-brom-2-fluor-5-isopropoxyphenyl)-4-trifluormethyl-6(1H)-pyrimidinon, Smp. 106-108°C;

-2-Chlor-5-[2-chlor-5-fluor-6-oxo-4-pentafluoräthyl-1(6H)-pyrimidinyl]-4-fluorbenzoesäure-isopropylester und Pyridin in Aethanol den 5-[2-Aethoxy-5-fluor-6-oxo-4-pentafluoräthyl-1(6H)-pyrimidinyl]-2-chlor-4-fluor-benzoesäure-isopropylester, $^1$H-NMR ($CDCl_3$, 400 MHz): 7,84 ppm (d,1H), 7,41 ppm (d,1H), 5,27 ppm (m,1H), 4,46 ppm (m,2H), 1,38 ppm (d,3H), 1,37 ppm (d,3H), 1,29 ppm (t,3H);

-2-Chlor-5-[2-chlor-5-fluor-6-oxo-4-pentafluoräthyl-1(6H)-pyrimidinyl]-4-fluorbenzoesäure-isopropylester und Pyridin in Propargylalkohol den 2-Chlor-5-[5-fluor-6-oxo-4-pentafluoräthyl-2-(2-propinyloxy)-1(6H)-pyrimidinyl]-4-fluorbenzoesäure-isopropylester, $^1$H-NMR ($CDCl_3$, 400 MHz): 7,86 ppm (d,1H), 7,43 ppm (d,1H), 5,27 ppm (m,1H), 4,97 ppm (d,2H), 2,53 ppm (t,1H), 1,39 ppm (d,3H), 1,38 ppm (d,3H);

-2-Chlor-1-(4-chlor-2-fluor-5-isopropoxyphenyl)-5-fluor-4-trifluormethyl-6(1H)-pyrimidinon und Natriummethylat in Methanol das 1-(4-Chlor-2-fluor-5-isopropoxyphenyl)-5-fluor-2-methoxy-4-trifluormethyl-6(1H)-pyrimidinon, Smp. 144-146°C;

-2-Chlor-5-[2-chlor-5-fluor-6-oxo-4-trifluormethyl-1(6H)-pyrimidinyl]-4-fluorbenzoesäure-isopropylester und Natriumisopropylat in Isopropanol den 2-Chlor-5-[5-fluor-2-isopropoxy-6-oxo-4-trifluormethyl-1(6H)-pyrimidinyl]-4-fluorbenzoesäure-isopropylester, $^1$H-NMR ($CDCl_3$, 400 MHz): 7,83 ppm (d,1H), 7,40 ppm (d,1H), 5,36 ppm (m,1H), 5,27 ppm (m,1H), 1,39 ppm (d,3H), 1,38 ppm (d,3H), 1,29 ppm (d,3H), 1,28 ppm (d.3H).

## Beispiel 3

Ein Gemisch von 9,2 g rohem 2-Chlor-5-[3,6-dihydro-2,6-dioxo-5-fluor-4-pentafluoräthyl -1(2H)-pyrimidinyl]-4-fluorbenzoesäure-isopropylester, 3,36 g Natriumhydrogencarbonat und 3,78 g Dimethylsulfat in 50 ml wasserfreiem Aceton wird 3 Stunden auf Rückflusstemperatur erhitzt. Nach Abkühlen werden die festen Anteile abgenutscht und das Filtrat unter vermindertem Druck zur Trockene eingedampft. der Rückstand wird an einer Kieselgel-Säule unter Verwendung von n-Hexan/Diäthyläther (2:1) chromatographisch aufgetrennt. Man erhält den 2-Chlor-4-fluor-5-[5-fluor-2-methoxy-6-oxo-4-pentafluoräthyl -1(6H)-pyrimidinyl]-benzoesäure-isopropylester, Smp. 75-77°C.

In analoger Weise erhält man beim Einsatz von:

-3-(4-Chlor-3-isopropoxyphenyl)-6-trifluormethyl-2,4(1H,3H)-pyrimidindion mit Dimethylsulfat und Natriumhydrogencarbonat in Aceton das 1-(4-Chlor-3-isopropoxyphenyl)-2-methoxy-4-trifluormethyl)-6(1H)-pyrimidinon, Smp. 55-58°C.

Beispiel 4

Zu einer Suspension von 0,93 g einer 55%igen Natriumhydrid-Dispersion in 25 ml Dimethylformamid werden bei Raumtemperatur unter Rühren 9,51 g 2-Chlor-5-[3,6-dihydro-2,6-dioxo-4-pentafluoräthyl -1(2H)-pyrimidinyl]-4-fluorbenzoesäure-isopropylester zugefügt, und das Gemisch wird 30 Minuten bei Raumtemperatur nachgerührt. Anschliessend wird mit 4,04 g Dimethylsulfat versetzt und 4 Stunden bei 50°C nachgerührt. Das Reaktionsgemisch wird abgekühlt und auf 500 ml Wasser gegossen und die wässrige Lösung wird zweimal mit je 100 ml Essigsäure-äthylester ausgeschüttelt. Die organische Phase wird zweimal mit je 100 ml Wasser gewaschen, über wasserfreiem Natriumsulfat getrocknet und unter vermindertem Druck zur Trockene eingedampft. Der Rückstand wird an einer Kieselgel-Säule unter Verwendung von n-Hexan/Diäthyläther (2:1) als Laufmittel chromatographisch aufgetrennt. Das Produkt wird aus n-Hexan umkristallisiert. Man erhält den 2-Chlor-4-fluor-5-[2-methoxy-6-oxo-4-pentafluoräthyl -1(6H)-pyrimidinyl]-benzoesäure-isopropylester, Smp. 82-84°C.

In analoger Weise erhält man beim Einsatz von

-3-(4-Chlor-2-fluor-5-isopropoxyphenyl)-6-trifluormethyl-2,4(1H,3H)-pyrimidindion das 1-(4-Chlor-2-fluor-5-iso propoxyphenyl)-2-methoxy-4-trifluormethyl-6(1H)-pyrimidinon, Smp. 117-119°C;

-3-(4-Chlor-2-fluor-5-isopropoxyphenyl)-6-pentafluoräthyl -2,4(1H,3H)-pyrimidindion mit Natriumhydrogencarbonat und Dimethylsulfat in Aceton das 1-(4-Chlor-2-fluor-5-isopropoxyphenyl)-2-methoxy-4-pentafluoräthyl-6(1H)-pyrimidinon, Smp. 79-81°C;

-3-(4-Chlor-2-fluor-5-isopropoxyphenyl)-5-fluor-6-pentafluoräthyl-2,4(1H,3H)-pyrimidindion mit Natriumhydrogencarbonat und Dimethylsulfat in Aceton das 1-(4-Chlor-2-fluor-5-isopropoxyphenyl)-5-fluor-2-methoxy-4-pentafluoräthyl-6(1H)-pyrimidinon, Smp. 69-71°C;

-2-Chlor-5-[3,6-dihydro-2,6-dioxo-4-trifluormethyl-1(2H)-pyrimidinyl]-4-fluorbenzoesäure-isopropylester den 2-Chlor-4-fluor-5-[2-methoxy-6-oxo-4-trifluormethyl-1(6H)-pyrimidinyl]-benzoesäure-isopropylester, Smp. 121-123°C;

-2-Chlor-5-[3,6-dihydro-2,6-dioxo-5-fluor-4-trifluormethyl-1(2H)-pyrimidinyl]-4-fluorbenzoesäure-isopropylester den 2-Chlor-4-fluor-5-[5-fluor-2-methoxy-6-oxo-4-trifluormethyl-1(6H)-pyrimidinyl]-benzoesäure-isopropylester, Smp. 104-106°C;

-2-Chlor-5-(1,2,4,5,6,7-hexahydro-2,4-dioxo -3H-cyclopenta[d]pyrimidin-3-yl)-benzoesäure-äthylester mit Natriumhydrid und Isopropylbromid in Hexamethyl-phosphorsäure-triamid den 2-Chlor-5-(4,5,6,7-tetrahydro-2-isopropoxy-4-oxo-3H-cyclopenta[d]pyrimidin-3-yl)-benzoesäure-äthylester. $^1$H-NMR ($CDCl_3$, 400 MHz): 7,69 ppm (d,1H), 7,54 ppm (d,1H), 7,23 ppm (q,1H), 5,30 ppm (m,1H), 4,38 ppm (q,2H), 2,79 ppm (m,4H), 2,09 ppm (m,2H), 1,38 ppm (t,3H), 1,22 ppm (d,6H).

Beispiel 5

Ein Gemisch von 2,0 g 1-(4-Chlor-2-fluor-5-hydroxyphenyl)-2-methoxy-4-trifluormethyl-6(1H)-pyrimidinon, 1,06 g Propargylbromid und 1,64 g Kaliumcarbonat in 50 ml wasserfreiem Aceton wird 2,5 Stunden unter Rühren bei dem Siedepunkt erhitzt. Anschliessend werden die unlöslichen Anteile abgenutscht, und das Filtrat wird unter vermindertem Druck zur Trockene eingedampft. Man löst den Rückstand in 100 ml Essigsäure-äthylester und schüttelt die Lösung dreimal mit je 50 ml Wasser aus. Dann wird die organische Phase über wasserfreiem Natriumsulfat getrocknet und zur Trockene eingedampft. Der Rückstand wird aus Diäthyläther/n-Hexan umkristallisiert. Man erhält das 1-(4-Chlor-2-fluor-5-propargyloxyphenyl) -2-methoxy-4-trifluormethyl-6(1H)-pyrimidinon, Smp. 108-110°C.

In analoger Weise erhält man beim Einsatz von:

-1-(4-Chlor-2-fluor-5-hydroxyphenyl)-2-methoxy-4-trifluormethyl-6(1H)-pyrimidinon und Chlorameisensäure-methylester mit Pyridin in Methylenchlorid das {2-Chlor-4-fluor-5-[2-methoxy-6-oxo-4-trifluormethyl-1(6H)-pyrimidinyl]-phenyl}methylcarbonat, $^1$H-NMR ($CDCl_3$, 400 MHz): 7,40 ppm (d,1H), 7,24 ppm (d,1H), 6,61 ppm (s,1H), 4,02 ppm (s,3H), 3,95 ppm (s,3H);

-1-(4-Chlor-2-fluor-5-hydroxyphenyl)-2-methoxy-4-trifluormethyl-6(1H)-pyrimidinon und Acetylchlorid mit Pyridin in Methylenchlorid das 2-Chlor-4-fluor-5-[2-methoxy-6-oxy-4-trifluormethyl-1(6H)-pyrimidinyl]-phenylacetat, Smp. 110-112°C;

-1-(4-Chlor-2-fluor-5-hydroxyphenyl)-2-methoxy-4-trifluormethyl-6(1H)-pyrimidinon und Methyljodid mit Natriumhydrid in Dimethylformamid das 1-(4-Chlor-2-fluor-5-methoxyphenyl)-2-methoxy-4-trifluormethyl-1-(6H)-pyrimidinon, Smp. 167-169°C;

-1-(4-Chlor-2-fluor-5-hydroxyphenyl)-2-methoxy-4-trifluormethyl-6(1H)-pyrimidinon und Chlordimethyläther mit Natriumcarbonat in Aceton das 1-[4-Chlor-2-fluor-5-(methoxymethoxy)-phenyl]-2-methoxy-4-trifluormethyl-6(1H)-pyrimidinon, Smp. 97-98°C;

-1-(4-Chlor-2-fluor-5-hydroxyphenyl)-2-methoxy-4-trifluormethyl-6(1H)-pyrimidinon und Allylbromid mit Natriumcarbonat in Aceton das 1-(5-Allyloxy-4-chlor-2-fluorphenyl)-2-methoxy-4-trifluormethyl-6(1H)-pyrimidinon, Smp. 103-104°C;

-1-(4-Chlor-2-fluor-5-hydroxyphenyl)-2-methoxy-4-pentafluoräthyl-6(1H)-pyrimidinon und Methoxyacetylchlorid mit Pyridin in Methylenchlorid das {2-Chlor-4-fluor-5-[2-methoxy-6-oxo-4-pentafluoräthyl-1(6H)-pyrimidinyl]-phenyl}-methoxyacetat, Smp. 130-132°C;

-1-(4-Chlor-2-fluor-5-hydroxyphenyl)-2-methoxy-4-pentafluoräthyl-6(1H)-pyrimidinon und Dimethylsulfat mit Natriumcarbonat in Aceton das 1-(4-Chlor-2-fluor-5-methoxyphenyl)-2-methoxy-4-pentafluoräthyl-6(1H)-pyrimidinon, Smp. 123-125°C;

-1-(4-Chlor-2-fluor-5-hydroxyphenyl)-2-methoxy-4-pentafluoräthyl-6(1H)-pyrimidinon und Allylbromid mit Natriumcarbonat in Aceton das 1-(5-Allyloxy-4-chlor-2-fluorphenyl)-2-methoxy-4-pentafluoräthyl-6(1H)-pyrimidinon, Smp. 82-83°C;

-1-(4-Chlor-2-fluor-5-hydroxyphenyl)-2-methoxy-4-pentafluoräthyl-6(1H)-pyrimidinon und Chlorameisensäure-propargylester und Pyridin in Methylenchlorid das {2-Chlor-4-fluor-5-[2-methoxy-6-oxo-4-pentafluoräthyl-1(6H)-pyrimidinyl]-phenyl}2-propinylcarbonat, $^1$H-NMR ($CDCl_3$, 400 MHz): 7,41 ppm (d,1H), 7,28 ppm (d,1H), 6,66 ppm (s,1H), 4,88 ppm (d,2H), 4,00 ppm (s,3H), 2,61 ppm (t,1H);

-1-(4-Chlor-2-fluor-5-hydroxyphenyl)-2-äthoxy-4-pentafluoräthyl-6(1H)-pyrimidinon und Dimethylsulfat mit Natriumcarbonat in Aceton das 2-Aethoxy-1-(4-chlor-2-fluor-5-methoxyphenyl)-4-pentafluoräthyl-6(1H)-pyrimidinon, $^1$H-NMR ($CDCl_3$, 400 MHz): 7,33 ppm (d,1H), 6,79 ppm (d,1H), 6,65 ppm (s,1H), 4,48 ppm (m,2H), 3,89 ppm (s,3H), 1,29 ppm (t,3H);

-1-(4-Chlor-2-fluor-5-hydroxyphenyl)-2-methoxy-4-pentafluoräthyl-6(1H)-pyrimidinon und Propargylbromid mit Natriumcarbonat in Aceton das 1-(4-Chlor-2-fluor-5-(2-propinyloxy)-phenyl]-2-methoxy-4-pentafluoräthyl-6(1H)-pyrimidinon, $^1$H-NMR ($CDCl_3$, 400 MHz): 7,35 ppm (d,1H), 6,98 ppm (d,1H), 6,67 ppm (s,1H), 4,77 ppm (m,2H), 3,99 ppm (s,3H), 2,58 ppm (t,1H);

-2-Aethoxy-1-(4-chlor-2-fluor-5-hydroxyphenyl)-4-trifluormethyl-6(1H)-pyrimidinon und Chlorameisensäure-(n-butyl)ester mit Pyridin in Diäthyläther das n-Butyl {5-[2-äthoxy-6-oxo-4-trifluormethyl-1(6H)-pyrimidinyl]-2-chlor-4-fluorphenyl}carbonat, Smp. 100-102°C;

-2-Aethoxy-1-(4-chlor-2-fluor-5-hydroxyphenyl)-4-trifluormethyl-6(1H)-pyrimidinon und Dimethylsulfat mit Natriumcarbonat in Aceton das 2-Aethoxy-1-(4-chlor-2-fluor-5-methoxyphenyl)-4-trifluormethyl-6(1H)-pyrimidinon, Smp. 117-119°C;

-2-Aethoxy-1-(4-chlor-2-fluor-5-hydroxyphenyl)-4-trifluormethyl-6(1H)-pyrimidinon und Propargylbromid mit Natriumcarbonat in Aceton das 2-Aethoxy-1-[4-chlor-2-fluor-5-(2-propinyloxy)-phenyl]-4-trifluormethyl-6-(1H)-pyrimidinon, $^1$H-NMR ($CDCl_3$, 400 MHz): 7,34 ppm (d,1H), 6,97 ppm (d,1H), 6,60 ppm (s,1H), 4,76 ppm (m,2H), 4,50 ppm (m,2H), 2,57 ppm (t,1H), 1,29 ppm (t,3H);

-1-(4-Chlor-2-fluor-5-hydroxyphenyl)-4-pentafluoräthyl-2-(n-propoxy)-6(1H)-pyrimidinon und Propargylbromid mit Natriumcarbonat in Aceton das 1-[4-Chlor-2-fluor-5-(2-propinyloxy)-phenyl]-4-pentafluoräthyl-2-(n-propoxy)-6(1H)-pyrimidinon, $^1$H-NMR ($CDCl_3$, 400 MHz): 7,34 ppm (d,1H), 6,98 ppm (d,1H), 6,65 ppm (s,1H), 4,76 ppm (m,2H), 4,36 ppm (m,2H), 2,56 ppm (t,1H), 1,67 ppm (m,2H), 0.85 ppm (t,3H);

-2-Aethoxy-1-(4-chlor-2-fluor-5-hydroxyphenyl)-4-pentafluoräthyl-6(1H)-pyrimidinon und Propargylbromid mit Natriumcarbonat in Aceton das 2-Aethoxy-1-[4-chlor-2-fluor-5-(2-propinyloxy)-phenyl]-4-pentafluoräthyl-6(1H)-pyrimidinon, $^1$H-NMR ($CDCl_3$, 400 MHz): 7,35 ppm (d,1H), 6,98 ppm (d,1H), 6,65 ppm (s,1H), 4,77 ppm (m,2H), 4,48 ppm (m,2H), 2,57 ppm (t,1H), 1,29 ppm (t,3H);

-2-Aethoxy-1-(4-chlor-2-fluor-5-hydroxyphenyl)-4-trifluormethyl-6(1H)-pyrimidinon und Isobutyrylchlorid mit Pyridin in Diäthyläther das {5-[2-Aethoxy-6-oxo-4-trifluormethyl-1(6H)-pyrimidinyl]-2-chlor-4-fluorphenyl}-isobutyrat, $^1$H-NMR ($CDCl_3$, 400 MHz): 7,38 ppm (d,1H), 7,13 ppm (d,1H), 6,58 ppm (s,1H), 4,50 ppm (m,2H), 2,87 ppm (m,1H), 1,35 ppm (d,3H), 1,34 ppm (d,3H), 1,31 ppm (t,3H);

-2-Aethoxy-1-(4-chlor-2-fluor-5-hydroxyphenyl)-4-pentafluoräthyl-6(1H)-pyrimidinon und Chlorameisensäure-2-methoxyäthylester mit Pyridin in Diäthyläther das {5-[2-Aethoxy-6-oxo-4-pentafluoräthyl-1(6H)-pyrimidinyl]-2-chlor-4-fluorphenyl}-2-methoxyäthylcarbonat, $^1$H-NMR ($CDCl_3$, 400 MHz): 7,39 ppm (d,1H), 7,26 ppm (d,1H), 6,64 ppm (s,1H), 4,39-4,56 ppm (m,4H), 3,70 ppm (m,2H), 3,43 ppm (s,3H), 1,30 ppm (t,3H);

-1-(4-Brom-2-fluor-5-hydroxyphenyl)-2-methoxy-4-trifluormethyl-6(1H)-pyrimidinon und Methyljodid mit Natriumhydrid in Dimethylformamid das 1-(4-Brom-2-fluor-5-methoxyphenyl)-2-methoxy-4-trifluormethyl-6-(1H)-pyrimidinon, Smp. 175-177°C;

-1-(4-Brom-2-fluor-5-hydroxyphenyl)-2-methoxy-4-trifluormethyl-6(1H)-pyrimidinon und Allylbromid mit Natriumhydrid in Dimethylformamid das 1-(5-Allyloxy-4-brom-2-fluorphenyl)-2-methoxy-4-trifluormethyl-6-(1H)-pyrimidinon, Smp. 107-110°C;

-1-(4-Brom-2-fluor-5-hydroxyphenyl)-2-methoxy-4-trifluormethyl-6(1H)-pyrimidinon und Propargylbromid mit Natriumhydrid in Dimethylformamid das 1-[4-Brom-2-fluor-5-(2-propinyloxy)-phenyl]-2-methoxy-4-trifluormethyl-6(1H)-pyrimidinon, Smp. 136-138°C;

-1-(4-Chlor-2-fluor-5-hydroxyphenyl)-5-fluor-2-methoxy-4-trifluormethyl-6(1H)-pyrimidinon und Acrylsäurechlorid mit Natriumhydrid in Dimethylformamid das {2-Chlor-4-fluor-5-[5-fluor-2-methoxy-6-oxo-4-trifluormethyl-1(6H)-pyrimidinyl]-phenyl}acrylat, $^1$H-NMR ($CDCl_3$, 400 MHz): 7,42 ppm (d,1H), 7,21 ppm (d,1H), 6,68 ppm (q,1H), 6,35 ppm (q,1H), 6,12 ppm (q,1H), 4,00 ppm (s,3H);

-1-(4-Chlor-2-fluor-5-hydroxyphenyl)-5-fluor-2-methoxy-4-trifluormethyl-6(1H)-pyrimidinon und Chlorameisensäure-allylester mit Pyridin in Diäthyläther das {2-Chlor-4-fluor-5-{5-fluor-2-methoxy-6-oxo-4-trifluormethyl-1(6H)-pyrimidinyl]-phenyl}-2-propenylcarbonat, $^1$H-NMR ($CDCl_3$, 400 MHz): 7,42 ppm (d,1H), 7,26 ppm (d,1H), 5,94-6,06 ppm (m,1H), 5,45 ppm (m,1H), 5,36 ppm (m,1H), 4,78 ppm (m,2H), 4,00 ppm (s,3H);

-1-(4-Chlor-2-fluor-5-hydroxyphenyl)-5-fluor-2-methoxy-4-pentafluoräthyl-6(1H)-pyrimidinon und Chlordimethyläther mit Natriumhydrid in Dimethylformamid das 1-(4-Chlor-2-fluor-5-methoxymethoxy-phenyl)-5-fluor-2-methoxy-4-pentafluoräthyl-6(1H)-pyrimidinon, $^1$H-NMR ($CDCl_3$, 400 MHz): 7,34 ppm (d,1H), 7,13 ppm (d,1H), 5,22 ppm (q,2H), 3,98 ppm (s,3H), 3,52 ppm (s,3H);

-1-(4-chlor-2-fluor-5-hydroxyphenyl)-5-fluor-2-methoxy-4-pentafluoräthyl-6(1H)-pyrimidinon und Crotonsäurechlorid mit Natriumhydrid in Dimethylformamid das {2-Chlor-4-fluor-5-[5-fluor-2-methoxy-6-oxo-4-pentafluoräthyl-1(6H)-pyrimidinyl]-phenyl}crotonat, $^1$H-NMR ($CDCl_3$, 400 MHz): 7,42 ppm (d,1H), 7,28 ppm (m,1H), 7,20 ppm (d,1H), 6,08 ppm (m,1H), 3,98 (s,3H), 2,01 ppm (q,3H);

-1-(4-Chlor-2-fluor-5-hydroxyphenyl)-2-propoxy-4-trifluormethyl-6(1H)-pyrimidinon und Propargylbromid mit Natriumcar-bonat in Aceton das 1-[4-Chlor-2-fluor-5-(2-propinyloxy)-phenyl]-2-propoxy-4-trifluormethyl-6-(1H)-pyrimidinon, $^1$H-NMR ($CDCl_3$, 400 MHz): 7,34 ppm (d,1H), 6.98 ppm (d,1H), 6,60 ppm (s,1H), 4,76 ppm (m,2H), 4,39 ppm (m,2H), 2,56 ppm (t,1H), 1,67 ppm (m,2H), 0,85 ppm (t,3H);

-2-Aethoxy-1-(4-brom-2-fluor-5-hydroxyphenyl)-4-trifluormethyl-6(1H)-pyrimidinon und Propargylbromid mit Natriumhydrid in Dimethylformamid das 2-Aethoxy-1-[4-brom-2-fluor-5-(2-propinyloxy)-phenyl]-4-trifluormethyl-6(1H)-pyrimidinon, Smp. 101-102°C;

-2-Aethoxy-1-(4-brom-2-fluor-5-hydroxyphenyl)-4-trifluormethyl-6(1H)-pyrimidinon und Allylbromid mit Natriumhydrid in Dimethylformamid das 2-Aethoxy-1-[4-brom-2-fluor-5-(2-propenyloxy)-phenyl]-4-trifluormethyl-6(1H)-pyrimidinon, Smp. 68-70°C;

-2-Aethoxy-1-(4-brom-2-fluor-5-hydroxyphenyl)-4-trifluormethyl-6(1H)-pyrimidinon und Methyljodid mit Natriumhydrid in Dimethylformamid das 2-Aethoxy-1-(4-brom-2-fluor-5-methoxyphenyl)-4-trifluormethyl-6-(1H)-pyrimidinon, Smp. 126-128°C.

## Beispiel 6

Zu einer Lösung von 2,10 g Trifluormethansulfonsäureanhydrid in 50 ml Diäthyläther wird unter Rühren bei 0°C eine Lösung von 0,80 g 2-[(Isopropylidenamino)oxy]-äthanol und 0,54 g Pyridin in 25 ml Diäthyläther während 15 Minuten zugetropft, und das Gemisch wird 15 Minuten bei 0°C nachgerührt. Die klare Lösung wird von unlöslichen Anteilen abpipettiert. Man erhält den Trifluormethansulfonsäure-{2-[-(isopropylidenamino)oxy]-äthyl}ester, der in der nächsten Stufe ohne Reinigung eingesetzt wird.

Zu einer Suspension von 0,22 g einer 55%-igen Natriumhydrid-Dispersion in 25 ml Dimethylformamid werden bei Raumtemperatur unter Rühren 1,70 g 1-(4-Chlor-2-fluor-5-hydroxyphenyl)-2-methoxy-4-trifluormethyl-6(1H)-pyrimidinon zugefügt, und das Gemisch wird 1 Stunde nachgerührt. Anschliessend wird die vorher zubereitete Lösung von Trifluormethansulfonsäure-{2-[(isopropylidenamino)oxy]-äthyl}ester bei 0°C zugefügt, und das Gemisch wird 30 Minuten nachgerührt. Man wäscht das Reaktionsgemisch zweimal mit je 100 ml Wasser, trocknet die organische Phase über wasserfreiem Natriumsulfat und dampft sie unter vermindertem Druck zur Trockene ein. Der Rückstand wird an einer Kieselgel-Säule unter Verwendung von n-Hexan/Diäthyläther (2:1) als Laufmittel chromatographisch gereinigt. Das Produkt wird aus n-Hexan/Diäthyläther umkristallisiert. Man erhält das 1-[4-Chlor-2-fluor-5-{2-[(isopropylidenamino)-oxy]-äthoxy}-phenyl]-2-methoxy-4-trifluormethyl-6(1H)pyrimidinon, Smp. 136-138°C.

In analoger Weise erhält man beim Einsatz von:

-2-Aethoxy-1-(4-brom-2-fluor-5-hydroxyphenyl)-4-trifluormethyl-6(1H)-pyrimidinon das 2-Aethoxy-1-[4-brom-2-fluor-5-{2-[(isopropylidenamino)oxy]-äthoxy}-phenyl]-4-trifluormethyl-6(1H)-pyrimidinon, Smp. 131-132°C;

-1-(4-Brom-2-fluor-5-hydroxyphenyl)-2-methoxy-4-trifluormethyl-6(1H)-pyrimidinon das 1-[4-Brom-2-fluor-5-{2-[(isopropylidenamino)oxy]-äthoxy}-phenyl]-2-methoxy-4-trifluormethyl-6(1H)-pyrimidinon, Smp. 149-150°C.

Beispiel 7

Zu einer Lösung von 1,60 g 1-(4-Chlor-2-fluor-5-hydroxyphenyl)-5-fluor-2-methoxy-4-pentafluoräthyl-6-(1H)-pyrimidinon, 0,30 g 4-Pentinsäure und 0,05 g 4-Pyrrolidino-pyridin in 50 ml Methylenchlorid wird unter Rühren bei Raumtempratur 0,74 g N,N'-Dicyclohexylcarbodiimid zugefügt, und das Gemisch wird 5 Stunden bei Raumtemperatur nachgerührt. Anschliessend werden unlösliche Anteile abgenutscht und das Filtrat zur Trockene eingedampft. Der Rückstand wird an einer Kieselgel-Säule unter Verwendung von n-Hexan/Diäthyläther (3:1) als Laufmittel chromatographisch gereinigt. Das Produkt wird aus n-Hexan/Diäthyläther umkristallisiert. Man erhält das 2-Chlor-4-fluor-5-[5-fluor-2-methoxy-6-oxo-4-pentafluoräthyl-1(6H)-pyrimidinyl]-phenyl-4-pentinoat, Smp. 107-109°C.

Beispiel 8

Eine Suspension von 1,90 g 2-Chlor-4-fluor-5-[2-methoxy-6-oxo-4-trifluormethyl-1(6H)-pyrimidinyl]-benzoesäure in 10 ml Benzol und 1,85 g Thionylchlorid wird unter Rühren 4 Stunden bis zur Bildung einer Lösung erhitzt. Das Reaktionsgemisch wird unter vermindertem Druck zur Trockene eingedampft, der Rückstand in 20 ml Methylenchlorid gelöst und die Lösung bei Raumtemperatur mit 5 ml n-Butanol und 0,50 g Pyridin unter Rühren versetzt. Das Reaktionsgemisch wird 3 Stunden nachgerührt und unter vermindertem Druck zur Trockene eingedampft. Man löst den Rückstand in 100 ml Essigsäure-äthylester, wäscht die Lösung dreimal mit je 50 ml Wasser, trocknet die organische Phase über wasserfreiem Natriumsulfat und dampft sie zur Trockene ein. Der harzige Rückstand wird an einer Kieselgel-Säule unter Verwendung von Diäthyläther/n-Hexan (1:3) als Laufmittel chromatographisch gereinigt. Man erhält den 2-Chlor-4-fluor-5-[2-methoxy-6-oxo-4-trifluormethyl-1(6H)-pyrimidinyl]-benzoesäure-(n-butyl)ester, $^1$H-NMR ($CDCl_3$: 400 MHz): 7,84 ppm (d,1H), 7,40 ppm (d,1H), 6,63 ppm (s,1H), 4,34 ppm (m,2H), 4,02 ppm (s,3H), 1,75 ppm (m,2H), 1,46 ppm (m,2H), 0,97 ppm (t,3H).

In analoger Weise erhält man beim Einsatz von:

-2-Chlor-4-fluor-5-[2-methoxy-6-oxo-4-trifluormethyl-1(6H)-pyrimidinyl]-benzoesäure und Thionylchlorid das Säurechlorid und aus diesem mit 2-Methoxyäthanol den 2-Chlor-4-fluor-5-[2-methoxy-6-oxo-4-trifluormethyl-1(6H)-pyrimidinyl]-benzoesäure-(2-methoxyäthyl)ester, Smp. 63-65°C;

-2-Chlor-4-fluor-5-[2-methoxy-6-oxo-4-trifluormethyl-1(6H)-pyrimidinyl]-benzoesäure und Thionylchlorid das Säurechlorid und aus diesem mit n-Propanol den 2-Chlor-4-fluor-5-[2-methoxy-6-oxo-4-trifluormethyl-1-(6H)-pyrimidinyl]-benzoesäure-(n-propyl)ester, Smp. 55-57°C;

-2-Chlor-4-fluor-5-[2-methoxy-6-oxo-4-trifluormethyl-1(6H)-pyrimidinyl]-benzoesäure und Thionylchlorid das Säurechlorid und aus diesem mit Aethanol den 2-Chlor-4-fluor-5-[2-methoxy-6-oxo-4-trifluormethyl-1-(6H)-pyrimidinyl]-benzoesäure-äthylester, Smp. 91-93°C;

-2-Chlor-4-fluor-5-[2-methoxy-6-oxo-4-trifluormethyl-1(6H)-pyrimidinyl]-benzoesäure und Thionylchlorid das Säurechlorid und aus diesem mit Methanol den 2-Chlor-4-fluor-5-[2-methoxy-6-oxo-4-trifluormethyl-1-(6H)-pyrimidinyl]-benzoesäure-methylester, Smp. 115-117°C;

-2-Chlor-4-fluor-5-[5-fluor-2-methoxy-6-oxo-4-trifluormethyl-1(6H)-pyrimidinyl]-benzoesäure und Thionylchlorid das Säurechlorid und aus diesem mit Aethanol den 2-Chlor-4-fluor-5-[5-fluor-2-methoxy-6-oxo-4-trifluormethyl-1(6H)-pyrimidinyl]benzoesäure-äthylester, Smp. 69-72°C;

-2-Chlor-4-fluor-5-[5-fluor-2-methoxy-6-oxo-4-trifluormethyl-1(6H)-pyrimidinyl]-benzoesäure und Thionylchlorid das Säurechlorid und aus diesem mit n-Propanol den 2-Chlor-4-fluor-5-[5-fluor-2-methoxy-6-oxo-4-trifluormethyl-1(6H)-pyrimidinyl]-benzoesäure-(n-propyl)ester, Smp. 76-78°C;

-2-Chlor-4-fluor-5-[5-fluor-2-methoxy-6-oxo-4-trifluormethyl-1(6H)-pyrimidinyl]-benzoesäure und Thionylchlorid das Säurechlorid und aus diesem mit n-Butanol den 2-Chlor-4-fluor-5-[5-fluor-2-methoxy-6-oxo-4-trifluormethyl-1(6H)-pyrimidinyl]-benzoesäure-(n-butyl)ester, $^1$H-NMR ($CDCl_3$, 400 MHz): 7,85 ppm (d,1H), 7,42 ppm (d,1H), 4,35 ppm (m,2H), 4,00 ppm (s,3H), 1,75 ppm (m,2H), 1,46 ppm (m,2H), 0,97 ppm (m,3H);

-2-Chlor-4-fluor-5-[5-fluor-2-methoxy-6-oxo-4-trifluormethyl-1(6H)-pyrimidinyl]-benzoesäure und Thionylchlorid das Säurechlorid und aus diesem mit 2-Methoxyäthanol den 2-Chlor-4-fluor-5-[5-fluor-2-methoxy-6-oxo-4-trifluormethyl-1(6H)-pyrimidinyl]-benzoesäure-(2-methoxyäthyl)ester, $^1$H-NMR ($CDCl_3$ , 400 MHz): 7,88 ppm (d,1H), 7,42 ppm (d,1H), 4,49 ppm (m,2H), 4,00 ppm (s,3H), 3,71 ppm (m,2H), 3,41 ppm (s,3H);

-2-Chlor-4-fluor-5-[5-fluor-2-methoxy-6-oxo-4-trifluormethyl-1(6H)-pyrimidinyl]-benzoesäure und Thionylchlorid das Säurechlorid und aus diesem mit Methanol den 2-Chlor-4-fluor-5-[5-fluor-2-methoxy-6-oxo-4-trifluormethyl-1(6H)-pyrimidinyl]-benzoesäure-methylester, $^1$H-NMR ($CDCl_3$, 400 MHz): 7,90 ppm (d,1H), 7,43 ppm (d,1H), 4,00 ppm (s,3H), 3,94 ppm (s,3H);

-2-Chlor-4-fluor-5-[2-methoxy-6-oxo-4-pentafluoräthyl-1(6H)-pyrimidinyl]-benzoesäure und Thionylchlorid das Säurechlorid und aus diesem mit Methanol den 2-Chlor-4-fluor-5-[2-methoxy-6-oxo-4-pentafluoräthyl-1-(6H)-pyrimidinyl]-bezoesäure-methylester, Smp. 80-82°C;

-2-Chlor-4-fluor-5-[2-methoxy-6-oxo-4-pentafluoräthyl-1(6H)-pyrimidinyl]-benzoesäure und Thionylchlorid das Säurechlorid und aus diesem mit Aethanol den 2-Chlor-4-fluor-5-[2-methoxy-6-oxo-4-pentafluoräthyl-1-(6H)-pyrimidinyl]-benzoesäure-äthylester, $^1$H-NMR ($CDCl_3$, 400 MHz): 7,87 ppm (d,1H), 7,40 ppm (d,1H), 6,67 ppm (s,1H), 4,40 ppm (m,2H), 4,00 ppm (s,3H), 1,40 ppm (t,3H);

-2-Chlor-4-fluor-5-[2-methoxy-6-oxo-4-pentafluoräthyl-1(6H)-pyrimidinyl]-benzoesäure und Thionylchlorid das Säurechlorid und aus diesem mit n-Propanol den 2-Chlor-4-fluor-5-[2-methoxy-6-oxo-4-pentafluoräthyl-1(6H)-pyrimidinyl]-benzoesäure-(n-propyl)ester, $^1$H-NMR ($CDCl_3$, 400 MHz): 7,86 ppm (d,1H), 7,40 ppm (d,1H), 6,67 ppm (s,1H), 4,30 ppm (m,2H), 4,00 ppm (s,3H), 1,80 ppm (m,2H), 1,02 ppm (t,3H);

-2-Chlor-4-fluor-5-[2-methoxy-6-oxo-4-pentafluoräthyl-1(6H)-pyrimidinyl]-benzoesäure und Thionylchlorid das Säurechlorid und aus diesem mit n-Butanol den 2-Chlor-4-fluor-5-[2-methoxy-6-oxo-4-pentafluoräthyl-1-(6H)-pyrimidinyl]-benzoesäure-(n-butyl)ester, $^1$H-NMR ($CDCl_3$, 400 MHz): 7,86 ppm (d,1H), 7,40 ppm (d,1H), 6,68 ppm (s,1H), 4,34 ppm (m,2H), 4,00 ppm (s,3H), 1,75 ppm (m,2H), 1,46 ppm (m,2H), 0,97 ppm (t,3H);

-2-Chlor-4-fluor-5-[2-methoxy-6-oxo-4-pentafluoräthyl-1(6H)-pyrimidinyl]benzoesäure und Thionylchlorid das Säurechlorid und aus diesem mit 2-Methoxyäthanol den 2-Chlor-4-fluor-5-[2-methoxy-6-oxo-4-pentafluoräthyl-1(6H)-pyrimidinyl]-benzoesäure-(2-methoxyäthyl)ester, Smp. 92-94°C;

-2-Chlor-4-fluor-5-[2-methoxy-6-oxo-4-trifluormethyl-1(6H)-pyrimidinyl]-benzoesäure und Thionylchlorid das Säurechlorid und aus diesem mit 2-[(Isopropylidenamino)oxy]-äthanol den 2-Chlor-4-fluor-5-[2-methoxy-6-oxo-4-trifluormethyl-1(6H)-pyrimidinyl]-benzoesäure-{2-[(isopropylidenamino)oxy]-äthyl}ester, $^1$H-NMR ($CDCl_3$, 400 MHz): 7,89 ppm (d,1H), 7,40 ppm (d,1H), 6,62 ppm (s,1H), 4,56 ppm (m,2H), 4,32 ppm (m,2H), 4,02 ppm (s,3H), 1,85 ppm (s,3H), 1,83 ppm (s,3H).

II. Herstellung der Ausgangsmaterialien der Formel I':

Beispiel 9

Eine Lösung von 3,55 g 3-Amino-4,4,4-trifluorcrotonsäure-äthylester in 50 ml n-Hexan wird unter Rühren bei 0-3°C während 15 Minuten zu 0,85 g einer 55%igen Natriumhydrid-Dispersion in 50 ml Dimethylformamid zugetropft, und das Gemisch wird 30 Minuten nachgerührt. Anschliessend wird während 5 Minuten unter Rühren und Kühlen bei -15°C eine Lösung von 5,0 g 2-Chlor-4-fluor-5-isocyanatobenzoesäure-isopropylester in 100 ml n-Hexan zugetropft. Die Temperatur des Reaktionsgemisches steigt auf 10°C an, und das Gemisch wird danach eine Stunde bei Raumtemperatur nachgerührt. Das dabei anfallende Zwischenprodukt 2-Chlor-4-fluor-5-[3-(2-äthoxycarbonyl -1-trifluormethyl-vinyl)ureido]-benzoesäure-isopropylester wird nicht isoliert.

Man bringt durch Zusatz von konzentrierter Essigsäure das Gemisch auf pH 4, giesst es auf 750 ml Wasser und extra hiert das wässrige Gemisch mit 300 ml Aethylacetat. Die organische Phase wird über wasserfreiem Natriumsulfat getrocknet und anschliessend unter vermindertem Druck zur Trockene eingedampft und der Rückstand aus Diäthyläther/ n-Hexan umkristallisiert. Man erhält auf diese Weise den 2-Chlor-5-[3,6-dihydro-2,6-dioxo-4-trifluormethyl -1(2H)-pyrimidinyl]-4-fluorbenzoesäure-isopropylester, Smp. 127-129°C.

In analoger Weise erhält man beim Einsatz von:

-3-Amino-4,4,5,5,5-pentafluor-2 -pentencarbonsäure-äthylester und 2-Chlor-4-fluor-5-isocyanatobenzoesäure-isopropylester über den 2-Chlor-5-[3-äthoxycarbonyl-1-pentafluoräthylvinyl)ureido] -4-fluor-benzoesäure-isopropylester den 2-Chlor-5-[3,6-dihydro-2,6-dioxo-4-pentafluoräthyl -1(2H)-pyrimidinyl]-4-fluorbenzoesäure-isopropylester, Smp. 189-191°C.

Beispiel 10

Eine Lösung von 1,0 g 3-Amino-4,4,4-trifluorcrotonsäure-äthylester in 10 ml absolutem Toluol wird unter Rühren bei 0°C während 15 Minuten zu einer Suspension von 0,24 g einer 55%igen Natriumhydrid-Dispersion in 20 ml absolutem Dimethylformamid zugetropft, und das Gemisch wird 15 Minuten bei 0°C nachgerührt. Anschliessend wird das Reaktionsgemisch auf -30°C abgekühlt und mit einer Lösung von 1,26 g 4-Chlor-2-fluor-5-isopropoxyphenylisocyanat in 10 ml absolutem Toluol versetzt. Die Temperatur steigt rasch auf -10°C an, und das Reaktiosgemisch wird danach 2 Stunden bei Raumtemperatur nachgerührt. Das Reaktionsgemisch wird auf eine Lösung von 3 ml 2N Salzsäure und 500 ml Wasser gegossen und das wässrige Gemisch zweimal mit je 100 ml Essigsäure-äthylester extrahiert, und die organischen Phasen werden zweimal mit je 50 ml Wasser gewaschen, über wasserfreiem Natriumsulfat getrocknet und unter vermindertem Druck zur Trockene eingedampft. Der Rückstand wird aus Diäthyläther/n-Hexan um kristallisiert. Man erhält das 3-(4-Chlor-2-fluor-5-isopropoxyphenyl)-6-trifluormethyl -2,4(1H,3H)-pyrimidindion, Smp. 195-197°C.

In analoger Weise erhält man beim Einsatz von:

-3-Amino-4,4,5,5,5-pentafluor-2 -pentencarbonsäure-äthylester und 4-Chlor-2-fluor-5-isopropoxyphenylisocyanat das 3-(4-Chlor-2-fluor-5-isopropoxyphenyl)-6-pentafluoräthyl -2,4(1H,3H)-pyrimidindion, Smp. 141-143°C;

-3-Amino-2,4,4,5,5,5-hexafluor-2 -pentencarbonsäure-äthylester und 4-Chlor-2-fluor-5-isopropoxyphenylisocyanat das 3-(4-Chlor-2-fluor-5-isopropoxyphenyl)-5-fluor -6-pentafluoräthyl-2,4(1H,3H)-pyrimidindion, Smp. 160-162°C.

Beispiel 11

In eine Lösung von 7,5 g 3-Oxo-2,4,4,4-tetrafluor-buttersäure-äthylester in 20 ml Toluol wird bei 75°C unter Rühren Ammoniak bis zur Sättigung eingeleitet. Anschliessend wird das Reaktionsgemisch unter Verwendung eines Wasserabscheiders 5 Stunden auf Rückflusstemperatur erhitzt, wobei das Zwischenprodukt 3-Amino-2,4,4,4-tetrafluorcrotonsäure-äthylester gebildet wird.

Das Reaktionsgemisch wird bei 0°C unter Rühren während 20 Minuten zu einer Suspension von 1,62 g einer 55%igen Natriumhydrid-Dispersion in 80 ml absolutem Dimethylformamid zugetropft und das Ganze 15 Minuten bei 0°C nachgerührt und danach auf -5°C abgekühlt. Man fügt eine Lösung von 9,56 g 2-Chlor-4-fluor-5-isocyanatobenzoesäure-isopropylester in 40 ml n-Hexan zu. Die Temperatur des Reaktionsgemisches steigt auf 10°C an, und das Gemisch wird danach 3 Stunden bei Raumtemperatur nachgerührt. Das dabei anfallende Zwischenprodukt 2-Chlor-4-fluor-5-[3-(2-äthoxycarbonyl-2-fluor -1-trifluormethyl-vinyl)-ureido]-benzoesäure-iso propylester wird nicht isoliert.

Das Reaktionsgemisch wird auf 1,5 l Wasser, das 20 ml 2N Salzsäure enthält, gegossen und das wässrige Gemisch zweimal mit je 200 ml Aethylacetat extrahiert. Man wäscht die organische Phase mit Wasser, trocknet sie über wasserfreiem Natriumsulfat und dampft sie unter vermindertem Druck zur Trockene ein. Der Rückstand wird mit 100 ml Diäthyläther und einer Lösung von 5 g Natriumhydrogencarbonat in 300 ml Wasser bei 50°C kurz gerührt und abgekühlt. Nach Abtrennen der wässrigen Phase wird die organische Phase dreimal mit einer Lösung von 2,5 g Natriumhydrogencarbonat in 100 ml Wasser ausgechüttelt, und anschliessend werden die vereinigten wässrigen Lösungen mit 15 ml konzentrierter Salzsäure auf pH 1 gestellt und mit Diäthyläther extrahiert. Dann wird die organische Phase mit Wasser gewaschen, über wasserfreiem Natriumsulfat getrocknet und unter vermindertem Druck zur Trockene eingedampft. Schliesslich wird der Rückstand aus Diäthyläther/n-Hexan umkristallisiert.

Auf diese Weise erhält man den 2-Chlor-5-[3,6-dihydro-2,6-dioxo-5-fluor -4-trifluormethyl-1(2H)-pyrimidinyl]-4-fluorbenzoesäure-isopropylester, Smp. 102-106°C.

Beispiel 12

In eine Lösung von 8,17 g 3-Aethoxy-2,4,4,5,5,5-hexafluor -3-hydroxypentancarbonsäure-äthylester in 30 ml Toluol wird während 20 Stunden unter Rühren ein schwacher Strom Ammoniak eingeleitet, zugleich wird ein azeotropisches Gemisch von Wasser, Aethanol und Toluol langsam abdestilliert. Es bildet sich als Zwischenprodukt der 3-Amino-2,4,4,5,5,5-hexafluor-2 -pentencarbonsäure-äthylester.

Das Reaktionsgemisch wird bei 0°C unter Rühren während 15 Minuten zu einer Suspension von 1,35 g einer 55%igen Natriumhydrid-Dispersion in 50 ml Dimethylformamid zuge tropft und das Ganze 15 Minuten bei 0°C nachgerührt und danach auf -30°C abgekühlt. Man fügt eine Lösung von 8,0 g 2-Chlor-4-fluor-5-isocyanatobenzoesäure-isopropylester in 30 ml Toluol zu. Die Temperatur des Reaktionsgemisches steigt auf -10°C an. Danach wird das Gemisch 3 Stunden bei Raumtemperatur nachgerührt. Das Reaktionsgemisch wird auf 1 l Wasser, das 17 ml 2N Salzsäure enthält, gegossen und das wässrige Gemisch zweimal mit je 200 ml Essigsäure-äthylester extrahiert. Man wäscht die organische Phase mit Wasser, trocknet sie über wasserfreiem Natriumsulfat und dampft sie unter vermindertem Druck zur Trockene ein. Der Rückstand wird mit 100 ml Diäthyläther und einer Lösung von 4,0 g Natriumhydrogencarbonat in 200 ml Wasser intensiv gerührt. Nach Abtrennen der wässrigen Phase wird die organische Phase dreimal mit einer Lösung von 2,5 g Natriumhydrogencarbonat in 100 ml Wasser ausgeschüttelt, und anschliessend werden die vereinigten wässrigen Lösungen mit 16 ml konzentrierter Salzsäure auf pH 1 gestellt und zweimal mit je 150 ml Diäthyläther ausgeschüttelt. Die organische Phase wird mit Wasser gewaschen, über wasserfreiem Natriumsulfat getrocknet und unter vermindertem Druck zur Trockene eingedampft.

Auf diese Weise erhält man den 2-Chlor-5-[3,6-dihydro-2,6-dioxo-5-fluor-4-pentafluoräthyl -1(2H)-pyrimidinyl]-4-fluorbenzoesäure-isopropylester, der ohne weitere Reinigung methyliert wird (siehe Beispiel 3).

III. Herstellung der Ausgangsmaterialien der Formel XI und I' (aus XI)

Beispiel 13

48,4 g 2-Chlor-5-ureido-benzoesäure-äthylester und 31,2 g Cyclopentanon-2-carbonsäure-äthylester werden in 500 ml Benzol und 2 g Toluol-4-sulfonsäure-Monohydrat 6 Stunden auf Rückflusstemperatur erhitzt. Das sich bildende Wasser wird mittels eines Wasserabscheiders entfernt. An schliessend wird das Reaktionsgemisch zur Trockene eingedampft, der Rückstand in 700 ml Diäthyläther gelöst und die Lösung filtriert. Das Filtrat wird zur Trockene eingedampft und der Rückstand an 1,5 kg Kieselgel unter Verwendung von Diäthyläther/n-Hexan (1:2) als Laufmittel chromatographisch gereinigt. Man erhält den 2-Chlor-5-[3-(2-äthoxycarbonyl-1-cyclopenten-1-yl)-ureido]-benzoesäure-äthylester als farblose Kristalle. Das Produkt wird aus Diäthyläther/n-Hexan umkristallisiert, Smp. 110-112°C.

Beispiel 14

Eine Lösung von 118,0 g 2-Chlor-5-[3-(2-äthoxycarbonyl-1-cyclopenten -1-yl)ureido]-benzoesäure-äthylester in 800 ml absolutem 1,2-Dimethoxyäthan wird unter Rühren bei 20°C während 10 Minuten zu einer Suspension von 7,7 g Natriumhydrid in 800 ml absolutem 1,2-Dimethoxyäthan zugetropft. Das Reaktionsgemisch wird anschliessend 1 Stunde nachgerührt, mit 20 ml Essigsäure versetzt und unter vermindertem Druck zur Trockene eingedampft. Der Rückstand wird in 2 l Methlyenchlorid gelöst und zweimal mit 1 l Wasser gewaschen. Die organische Phase wird über wasserfreiem Natriumsulfat getrocknet und bis zur Kristallisation eingedampft. Der Rückstand wird mit 1 l n-Hexan versetzt, und die Kristalle werden abgenutscht und mit n-Hexan nachgewaschen. Man erhält den 2-Chlor-5-(1,2,4,5,6,7-hexahydro-2,4-dioxo-3H-cyclopenta[d]pyrimidin-3-yl)-benzoesäure-äthylester, Smp. 178-180°C.

IV. Herstellung der Ausgangsmaterialien/Wirkstoffe der Formel IVa:

Beispiel 15

Zu einer Lösung von 3,80 g 1-(4-Chlor-2-fluor-5-isopropoxyphenyl) -2-methoxy-4-trifluormethyl-6(1H)-pyrimidinon (siehe Beispiel 4, 2. Teil) in 10 ml Methylenchlorid werden unter Rühren und Kühlen bei Raumtemperatur 3,70 ml konzentrierte Schwefelsäure während 1 Minute zugetropft. Das Reaktionsgemisch wird 3 Stunden bei Raumtemperatur nachgerührt und auf 15 g Eis gegossen. Die organische Phase wird abgetrennt und die wässrige Phase zweimal mit je 5 ml Methylenchlorid ausgeschüttelt, und die vereinten

organischen Phasen werden zweimal mit je 10 ml Wasser gewaschen. Anschliessend wird die Methylenchloridlösung über wasserfreiem Natriumsulfat getrocknet und unter vermindertem Druck zur Trockene eingedampft. Der Rückstand wird aus Diäthyläther/n-Hexan umkristallisiert. Man erhält das 1-(4-Chlor-2-fluor-5-hydroxyphenyl) -2-methoxy-4-trifluormethyl-6(1H)-pyrimidinon, Smp. 147-149°C.

In analoger Weise erhält man beim Einsatz von:

-1-(4-Chlor-2-fluor-5-isopropoxyphenyl)-4-pentafluoräthyl-2-propoxy-6(1H)-pyrimidinon mit konzentrierter Schwefelsäure bei 0°C während 1,5 Minuten das 1-(4-Chlor-2-fluor-5-hydroxyphenyl)-4-pentafluoräthyl-2-propoxy-6(1H)-pyrimidinon, Smp. 109-111°C;

-2-Aethoxy-1-(4-chlor-2-fluor-5-isopropoxyphenyl)-4-pentafluoräthyl-6(1H)-pyrimidinon mit konzentrierter Schwefelsäure bei 0°C während 1,5 Minuten das 2-Aethoxy-1-(4-chlor-2-fluor-5-hydroxyphenyl)-4-pentafluoräthyl-6(1H)-pyrimidinon, Smp. 117-120°C;

-1-(4-Chlor-2-fluor-5-isopropoxyphenyl)-2-methoxy-4-pentafluoräthyl-6(1H)-pyrimidinon mit konzentrierter Schwefelsäure bei Raumtemperatur während 20 Minuten das 1-(4-Chlor-2-fluor-5-hydroxyphenyl)-2-methoxy-4-pentafluoräthyl-6(1H)-pyrimidinon, Smp. 143-145°C;

-1-(4-Brom-2-fluor-5-isopropoxyphenyl)-2-methoxy-4-trifluormethyl-6(1H)-pyrimidinon mit konzentrierter Schwefelsäure das 1-(4-Brom-2-fluor-5-hydroxyphenyl)-2-methoxy-4-trifluormethyl-6(1H)-pyrimidinon, Smp. 132-136°C;

-1-(4-Chlor-2-fluor-5-isopropoxyphenyl)-2-propoxy-4-trifluormethyl-6(1H)-pyrimidinon mit konzentrierter Schwefelsäure während 2 Minuten bei 0°C das 1-(4-Chlor-2-fluor-5-hydroxyphenyl)-2-propoxy-4-trifluormethyl-6(1H)-pyrimidinon, Smp. 122-123°C;

-1-(4-Chlor-2-fluor-5-isopropoxyphenyl)-5-fluor-2-methoxy-4-trifluormethyl-6(1H)-pyrimidinon mit konzentrierter Schwefelsäure während 2 Minuten bei 0°C das 1-(4-Chlor-2-fluor-5-hydroxyphenyl)-5-fluor-2-methoxy-4-trifluormethyl -6(1H)-pyrimidinon, $^{1}$H-NMR ($CDCl_3$, 400 MHz): 7,30 ppm (d,1H), 6,87 ppm (d,1H), 4,00 ppm (s,3H);

-1-(4-Chlor-2-fluor-5-isopropoxyphenyl)-5-fluor-2-methoxy-4-pentafluoräthyl-6(1H)-pyrimidinon mit konzentrierter Schwefelsäure während 2 Minuten bei 0°C das 1-(4-Chlor-2-fluor-5-hydroxyphenyl)-5-fluor-2-methoxy-4-pentafluoräthyl-6(1H)-pyrimidinon, $^{1}$H-NMR ($CDCl_3$, 60 MHz): 7,28 ppm (d,1H), 6,83 ppm (d,1H), 5,10 ppm (s,1H), 4,00 ppm (s,3H);

-2-Aethoxy-1-(4-brom-2-fluor-5-isopropoxyphenyl)-4-trifluormethyl-6(1H)-pyrimidinon mit konzentrierter Schwefelsäure während 2 Minuten bei 0°C das 2-Aethoxy-1-(4-brom-2-fluor-5-hydroxyphenyl)-4-trifluormethyl-6(1H)-pyrimidinon, Smp. 160-161°C.

V. Herstellung der Ausgangsmaterialien der Formel IIa:

Beispiel 16

Zu einer Suspension von 38,8 g 2-Chlor-5-[3,6-dihydro-2,6-dioxo-4-trifluormethyl-1(2H)-pyrimidinyl]-4-fluorbenzoesäure-isopropylester in 45,2 g Phosphoroxychlorid und 40 ml Toluol werden bei Raumtemperatur unter Rühren und Kühlen 23,3 g Pyridin zugefügt. Es bildet sich rasch eine Lösung. Die Temperatur wird zwischen 30 bis 35°C gehalten, und nach 15 Minuten beginnt sich ein farbloser Niederschlag abzuscheiden. Man rührt das Reaktionsgemisch 45 Minuten bei 30-35°C nach und giesst es dann auf 300 g Eis. Anschliessend wird mit 200 ml Essigsäure-äthylester ausgeschüttelt, und die organische Phase wird dreimal mit je 50 ml Wasser gewaschen, über wasserfreiem Natriumsulfat getrocknet und unter vermindertem Druck zur Trockene eingedampft. Der Rückstand wird aus n-Hexan umkristallisiert. Man erhält den 2-Chlor-5-[2-chlor-6-oxo-4-trifluormethyl-1(6H)-pyrimidinyl]-4-fluorbenzoesäure-isopropylester, Smp. 72-75°C.

In analoger Weise erhält an beim Einsatz von:

-3-(4-Chlor-2-fluor-5-isopropoxyphenyl)-6-trifluormethyl-2,4(1H,3H)-pyrimidindion mit Phosphoroxychlorid und Pyridin innert 20 Minuten bei 30-35°C, danach 1 Stunde bei 70°C das 2-Chlor-1-(4-chlor-2-fluor-5-isopropoxyphenyl)-4-trifluormethyl-6(1H)-pyrimidinon, Smp. 101-103°C;

-2-Chlor-5-[3,6-dihydro-2,6-dioxo-4-trifluormethyl-1(2H)-pyrimidinyl]-4-fluorbenzoesäure-äthylester mit Phosphoroxychlorid und Pyridin innert 19 Stunden bei Raumtemperatur den 2-Chlor-5-[2-chlor-6-oxo-4-trifluormethyl-1(6H)-pyrimidinyl]-4-fluorbenzoesäure-äthylester, Smp. 88-90°C;

-2-Chlor-5-[3,6-dihydro-2,6-dioxo-4-pentafluoräthyl-1(2H)-pyrimidinyl]-4-fluorbenzoesäure-isopropylester mit Phosphoroxychlorid und Pyridin innert 1 Stunde bei Raumtemperatur den 2-Chlor-5-[2-chlor-6-oxo-4-pentafluoräthyl-1(6H)-pyrimidinyl]-4-fluorbenzoesäure-isopropylester, $^{1}$H-NMR ($CDCl_3$, 60 MHz): 7,90 ppm (dm,1H), 7,48 ppm (d,1H), 6,96 ppm (s,1H), 5,30 ppm (m,1H), 1,40 ppm (d,6H);

-3-(4-Chlor-2-fluor-5-isopropoxyphenyl)-6-pentafluoräthyl-2,4(1H,3H)-pyrimidinon mit Phosphoroxychlorid

und Pyridin innert 30 Minuten bei 0-30°C das 2-Chlor-1-(4-chlor-2-fluor-5-isopropoxyphenyl)-4-pentafluoräthyl-6(1H)-pyrimidinon, Smp. 55-58°C;

-2-Chlor-5-[3,6-dihydro-2,6-dioxo-4-trifluormethyl-1(2H)-pyrimidinyl]-4-fluorbenzoesäure-methylester mit Phosphoroxychlorid und Pyridin innert 7 Stunden bei Raumtemperatur den 2-Chlor-5-[2-chlor-6-oxo-4-trifluormethyl-1(6H)-pyrimidinyl]-4-fluorbenzoesäure-methylester, Smp. 89-91°C;

-2-Chlor-5-[3,6-dihydro-2,6-dioxo-5-fluor-4-trifluormethyl-1(2H)-pyrimidinyl]-4-fluorbenzoesäure-isopropylester mit Phosphoroxychlorid und Pyridin innert 1 Stunde bei Raumtemperatur, danach 1 Stunde bei 60°C den 2-Chlor-5-[2-chlor-5-fluor-6-oxo-4-trifluormethyl-1(6H)-pyrimidinyl]-4-fluorbenzoesäure-isopropylester, $^1$H-NMR ($CDCl_3$, 60 MHz): 7,88 ppm (d,1H), 7,47 ppm (d,1H), 5,30 ppm (m,1H), 1,41 ppm (d,6H);

-3-(4-Brom-2-fluor-5-isopropoxyphenyl)-6-trifluormethyl-2,4(1H,3H)-pyrimidindion mit Phosphoroxychlorid und Pyridin das 1-(4-Brom-2-fluor-5-isopropoxyphenyl)-2-chlor-4-trifluormethyl-6(1H)-pyrimidinon, Smp. 102°C;

-3-(4-Chlor-2-fluor-5-isopropoxyphenyl)-5-fluor-6-trifluormethyl-2,4(1H,3H)-pyrimidindion mit Phosphoroxychlorid und Pyridin innert 15 Minuten bei 20-40°C, danach 1 Stunde bei 70°C das 2-Chlor-1-(4-chlor-2-fluor-5-isopropoxyphenyl)-5-fluor-4-trifluormethyl-6(1H)-pyrimidinon, $^1$H-NMR ($CDCl_3$, 60 MHz): 7,42 ppm (d,1H), 6,84 ppm (d,1H), 4,50 ppm (m,1H), 1,44 ppm (d,6H);

-2-Chlor-5-[3,6-dihydro-2,6-dioxo-5-fluor-4-pentafluoräthyl-1(2H)-pyrimidinyl]-4-fluorbenzoesäure-isopropylester mit Phosphoroxychlorid und Pyridin innert 6 Stunden bei Raumtemperatur den 2-Chlor-5-[2-chlor-5-fluor-6-oxo-4-pentafluoräthyl-1(6H)-pyrimidinyl]-4-fluorbenzoesäure-isopropylester, $^1$H-NMR ($CDCl_3$, 400 MHz): 7,88 ppm (d,1H), 7,48 ppm (d,1H), 5,28 ppm (m,1H), 1,39 ppm (d,6H).

VI. Herstellung der Ausgangsmaterialien der Formel Va:

Beispiel 17

Eine Lösung von 5 g 2-Chlor-4-fluor-5-[2-methoxy-6-oxo-4-trifluormethyl -1(6H)-pyrimidinyl]-benzoesäure-isopropylester in 20 ml Methylenchlorid wird unter Rühren und Kühlen bei 20-25°C mit 25 ml konzentrierter Schwefelsäure versetzt. Das Reaktionsgemisch wird 20 Minuten bei Raumtemperatur nachgerührt und auf 100 g Eis gegossen. Die organische Phase wird abgetrennt, die wässrige Phase zweimal mit je 15 ml Aethylacetat ausgeschüttelt und die vereinigten organischen Phasen mit Wasser neutral gewaschen. Anschliessend wird die Lösung über wasserfreiem Natriumsulfat getrocknet und unter vermindertem Druck zur Trockene eingedampft. Der harzige Rückstand wird aus Diäthyläther/n-Hexan umkristallisiert. Man erhält die 2-Chlor-4-fluor-5-[2-methoxy-6-oxo-4-trifluormethyl -1(6H)-pyrimidinyl]-benzoesäure, Smp. 205-210°C.

In analoger Weise enthält man beim Einsatz von:

-2-Chlor-4-fluor-5-[2-methoxy-6-oxo-4-pentafluoräthyl -1(6H)-pyrimidinyl]-benzoesäure-isopropylester die 2-Chlor-4-fluor-5-[2-methoxy-6-oxo-4-pentafluoräthyl -1(6H)-pyrimidinyl]-benzoesäure, Smp. 197-199°C;

-2-Chlor-4-fluor-5-[5-fluor-2-methoxy-6-oxo -4-trifluormethyl-1(6H)-pyrimidinyl] -benzoesäure-isopropylester die 2-Chlor-4-fluor-5-[5-fluor-2-methoxy-6-oxo-4-trifluormethyl -1(6H)-pyrimidinyl]-benzoesäure, Smp. 199-201°C.

VI. Formulierungsbeispiele:

Beispiel 18

Ein emulgierbares Konzentrat enthält folgende Bestandteile:

| | |
|---|---|
| Verbindung der Formel Ia, Ib, IVa oder IVb (Wirkstoff) | 50 g/l |
| N-Methylpyrrolidon (Hilfslösungsmittel) | 200 g/l |
| Nonylphenol-(10)äthoxylat (nichtionogener Emulgator) | 50 g/l |
| Calcium-dodecylbenzolsulfonat (anionischer Emulgator) | 25 g/l |
| Gemisch von Alkylbenzolen (Lösungsmittel) | ad 1000 ml |

Der Wirkstoff und die Emulgatoren werden unter Rühren im Hilfslösungsmittel gelöst, und die Lösung wird mit dem Lösungsmittel auf 1 Liter ergänzt.

Das resultierende emulgierbare Konzentrat lässt sich in Wasser emulgieren und ergibt so eine gebrauchsfertige Spritzbrühe mit der gewünschten Konzentration.

Beispiel 19

Zur Herstellung eines 25% Spritzpulvers werden die nachstehend aufgeführten Bestandteile miteinander vermischt:

| | |
|---|---|
| Verbindung der Formel Ia, Ib, IVa oder IVb (Wirkstoff) | 25 g |
| Kieselsäure, hydratisiert | |
| (Trägerstoff, Mahlhilfsmittel) | 5 g |
| Natrium-laurylsulfat (Netzmittel) | 1 g |
| Natrium-lignosulfonat (Dispergator) | 2 g |
| Kaolin (Trägerstoff) | 67 g |
| | 100 g |

Anschliessend wird das Gemisch unter Verwendung einer Stiftmühle oder vergleichbaren Mahleinrichtung fein gemahlen.

Das resultierende Spritzpulver ergibt beim Einrühren in Wasser eine feine Suspension, die sich als gebrauchsfertige Spritzbrühe eignet.

## Ansprüche

1. $C_{1-4}$-Alkyl-, $C_{2-4}$-Alkenyl-und $C_{3\ \mathrm{oder}\ 4}$-Alkinyl-enoläther von 3-Aryluracilen der allgemeinen Formel

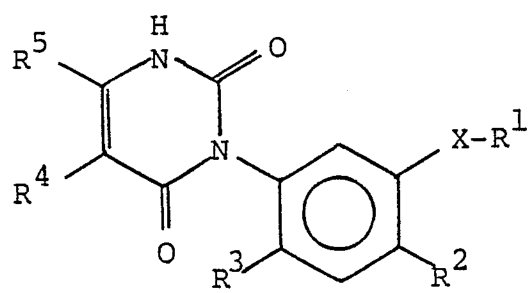

I'

worin

$R^1$ $C_{1-8}$-Alkyl, $C_{2-6}$-Alkenyl, $C_{2-6}$-Alkinyl, $C_{2-8}$ -Alkoxyalkyl oder eine Gruppe

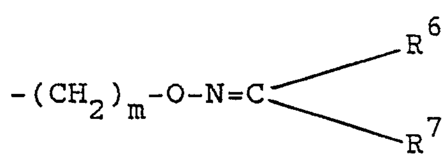

$R^2$ Halogen oder Cyano,
$R^3$ Wasserstoff oder Halogen,
$R^4$ Wasserstoff, Fluor oder $C_{1-4}$-Alkyl,
$R^5$ $C_{1-4}$-Alkyl oder $C_{1-4}$-Halogenalkyl,

oder

$R^4$ und $R^5$ zusammen Tri-oder Tetramethylen,
$R^6$ und $R^7$ unabhängig voneinander $C_{1-4}$-Alkyl,
m 1 oder 2

und

X O, O-C(O), O-C(O)-O oder C(O)-O bedeuten.

2. Verbindungen nach Anspruch 1, der allgemeinen Formel

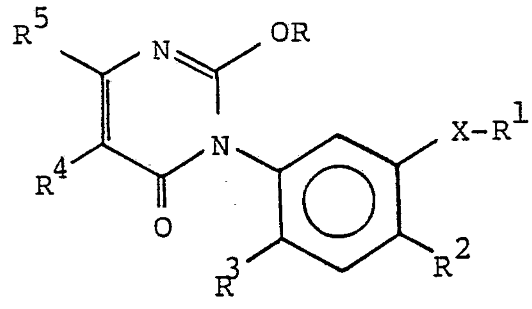

Ia

worin

$R^1$-$R^5$ und X die in Anspruch 1 angegebenen Bedeutungen besitzen

und

R $C_{1-4}$-Alkyl, $C_{2-4}$-Alkenyl oder $C_{3\ oder\ 4}$-Alkinyl bedeutet.

3. Verbindungen nach Anspruch 1, der allgemeinen Formel

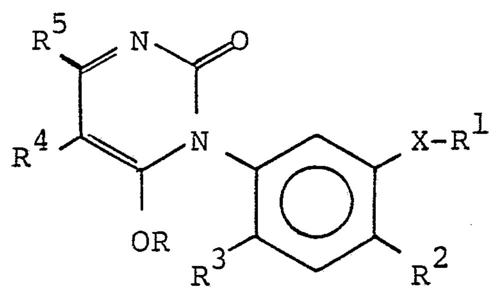

Ib

worin

$R^1$-$R^5$ und X die in Anspruch 1 angegebenen Bedeutungen besitzen

und

R $C_{1-4}$-Alkyl, $C_{2-4}$-Alkenyl oder $C_{3\ oder\ 4}$-Alkinyl bedeutet.

4. Verbindungen nach Anspruch 2 oder 3, worin R $C_{1-4}$-Alkyl, $C_{3\ oder\ 4}$-Alkenyl oder $C_{3\ oder\ 4}$-Alkinyl, $R^1$ $C_{1-6}$-Alkyl, $C_{3-6}$-Alkenyl, $C_{3-6}$-Alkinyl, $C_{2-7}$-Alkoxyalkyl oder eine Gruppe

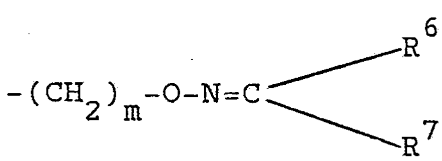

und $R^2$ Halogen bedeuten.

5. Verbindungen nach einem der Ansprüche 1 bis 4, worin $R^2$ Chlor oder Brom bedeutet.

6. Verbindungen nach einem der Ansprüche 1 bis 5, worin $R^3$ Fluor bedeutet.

7. Verbindungen nach einem der Ansprüche 1 bis 6, worin $R^5$ Trifluormethyl bedeutet.

8. Verbindungen nach einem der Ansprüche 1 bis 6, worin $R^5$ Pentafluoräthyl bedeutet.

9. Eine Verbindung nach Anspruch 1, ausgewählt aus:

2-Chlor-4-fluor-5-[2-methoxy-6-oxo-4-pentafluoräthyl-1(6H)-pyrimidinyl]-benzoesäure-isopropylester,

2-Chlor-4-fluor-5-[5-fluor-2-methoxy-6-oxo-4-pentafluoräthyl-1(6H)-pyrimidinyl]-benzoesäure-isopropylester,

1-(4-Chlor-2-fluor-5-isopropoxyphenyl)-2-methoxy-4-trifluormethyl-6(1H)-pyrimidinon,

1-(4-Chlor-2-fluor-5-isopropoxyphenyl)-2-methoxy-4-pentafluoräthyl-6(1H)-pyrimidinon,

1-(4-Chlor-2-fluor-5-isopropoxyphenyl)-5-fluor-2-methoxy-4-pentafluoräthyl-6(1H)-pyrimidinon,
2-Chlor-4-fluor-5-[2-methoxy-6-oxo-4-trifluormethyl-1(6H)-pyrimidinyl]-benzoesäure-isopropylester,
2-Chlor-4-fluor-5-[5-fluor-2-methoxy-6-oxo-4-trifluormethyl-1(6H)-pyrimidinyl]-benzoesäure-isopropylester,
1-(4-Chlor-2-fluor-5-propargyloxyphenyl)-2-methoxy-4-trifluormethyl-6(1H)-pyrimidinon,
2-Chlor-4-fluor-5-[2-methoxy-6-oxo-4-pentafluoräthyl-1(6H)-pyrimidinyl]-benzoesäure-methylester,
2-Chlor-4-fluor-5-[2-methoxy-6-oxo-4-pentafluoräthyl-1(6H)-pyrimidinyl]-benzoesäure-äthylester,
2-Chlor-4-fluor-5-[2-methoxy-6-oxo-4-pentafluoräthyl-1(6H)-pyrimidinyl]-benzoesäure-(n-propyl)ester und
2-Chlor-4-fluor-5-[2-methoxy-6-oxo-4-pentafluoräthyl-1(6H)-pyrimidinyl]-benzoesäure-(n-butyl)ester.

10. Eine Verbindung nach Anspruch 1, 2-Chlor-4-fluor-5-[2-methoxy-6-oxo-4-pentafluoräthyl-1(6H)-pyrimidinyl]-benzoesäure-2-methoxyäthylester.

11. Verbindungen der allgemeinen Formel

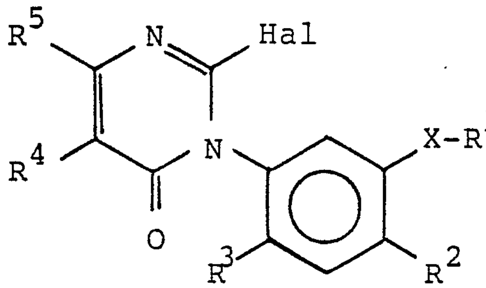

bzw.

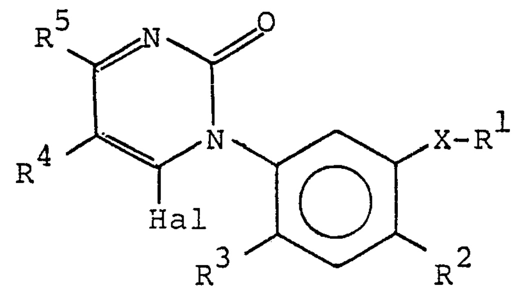

IIa IIb

worin

$R^1$ $C_{1-8}$-Alkyl, $C_{2-6}$-Alkenyl, $C_{2-6}$-Alkinyl, $C_{2-8}$-Alkoxyalkyl oder eine Gruppe

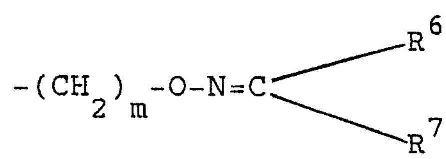

$R^2$ Halogen oder Cyano,
$R^3$ Wasserstoff oder Halogen,
$R^4$ Wasserstoff, Fluor oder $C_{1-4}$-Alkyl,
$R^5$ $C_{1-4}$-Alkyl oder $C_{1-4}$-Halogenalkyl,
oder
$R^4$ und $R^5$ zusammen Tri-oder Tetramethylen,
$R^6$ und $R^7$ unabhängig voneinander $C_{1-4}$-Alkyl,
m 1 oder 2,
X O, O-C(O), O-C(O)-O oder C(O)-O
und
Hal Chlor oder Brom bedeuten.

12. Verbindungen nach Anspruch 11, worin $R^1$ $C_{1-6}$-Alkyl, $C_{3-6}$-Alkenyl, $C_{3-6}$-Alkinyl, $C_{2-7}$-Alkoxyalkyl oder eine Gruppe

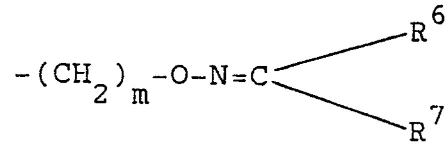

und $R^2$ Halogen bedeuten.

13. Verbindungen der allgemeinen Formel

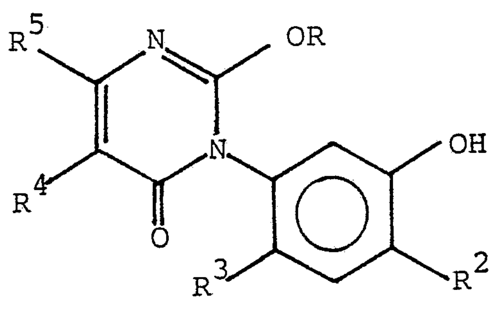

IVa

bzw.

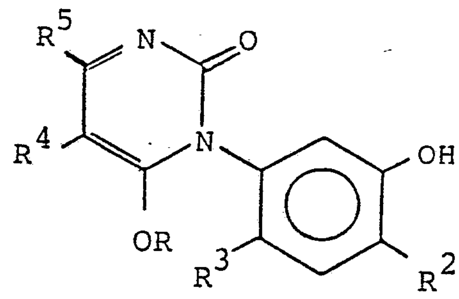

IVb

worin

R $C_{1-4}$-Alkyl, $C_{2-4}$-Alkenyl oder $C_{3\ \text{oder}\ 4}$-Alkinyl,
$R^2$ Halogen oder Cyano,
$R^3$ Wasserstoff oder Halogen,
$R^4$ Wasserstoff, Fluor oder $C_{1-4}$-Alkyl und
$R^5$ $C_{1-4}$-Alkyl oder $C_{1-4}$-Halogenalkyl,

oder

$R^4$ und $R^5$ zuammen Tri-oder Tetramethylen bedeuten.

14. Verbindungen der Formel IVa nach Anspruch 13, worin R $C_{1-4}$-Alkyl, $C_{3\ \text{oder}\ 4}$-Alkenyl oder $C_{3\ \text{oder}\ 4}$-Alkinyl und $R^2$ Halogen bedeuten.

15. Unkrautbekämpfungsmittel, dadurch gekennzeichnet, dass es eine wirksame Menge mindestens eines $C_{1-4}$-Alkyl-, $C_{2-4}$-Alkenyl-oder $C_{3\ \text{oder}\ 4}$-Alkinyl-enoläthers von 3-Aryluracilen der allgemeinen Formel

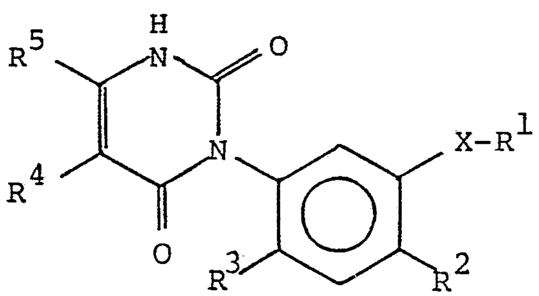

I'

worin

$R^1$ $C_{1-8}$-Alkyl, $C_{2-6}$-Alkenyl, $C_{2-6}$ -Alkinyl, $C_{2-8}$-Alkoxyalkyl oder eine Gruppe

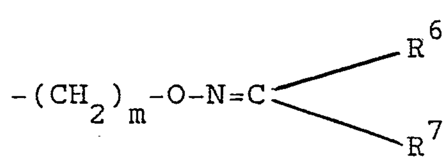

$R^2$ Halogen oder Cyano,
$R^3$ Wasserstoff oder Halogen,
$R^4$ Wasserstoff, Fluor oder $C_{1-4}$-Alkyl,
$R^5$ $C_{1-4}$-Alkyl oder $C_{1-4}$-Halogenalkyl,

oder

$R^4$ und $R^5$ zusammen Tri-oder Tetramethylen,
$R^6$ und $R^7$ unabhängig voneinander $C_{1-4}$-Alkyl,
m 1 oder 2

und

X O, O-C(O), O-C(O)-O oder C(O)-O bedeuten,
sowie Formulierungshilfsstoffe enthält.

16. Unkrautbekämpfungsmittel nach Anspruch 15, dadurch gekennzeichnet, dass es eine wirksame Menge mindestens eines $C_{1-4}$-Alkyl-, $C_{3\ \text{oder}\ 4}$-Alkenyl-oder $C_{3\ \text{oder}\ 4}$-Alkinyl-enoläthers von 3-Aryluracilen der Formel I', worin $R^1$ $C_{1-6}$-Alkyl, $C_{3-6}$-Alkenyl, $C_{3-6}$-Alkinyl, $C_{2-7}$-Alkoxyalkyl oder eine Gruppe

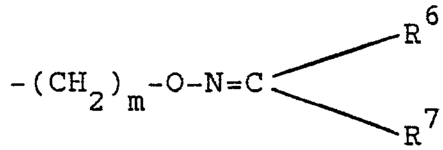

und $R^2$ Halogen bedeuten, sowie Formulierungshilfsstoffe enthält.

17. Unkrautbekämpfungsmittel nach Anspruch 15, dadurch gekennzeichnet, dass es eine wirksame Menge mindestens einer aus der Gruppe

2-Chlor-4-fluor-5-[2-methoxy-6-oxo-4-pentafluoräthyl-1(6H)-pyrimidinyl]-benzoesäure-isopropylester,
2-Chlor-4-fluor-5-[5-fluor-2-methoxy-6-oxo-4-pentafluoräthyl-1(6H)-pyrimidinyl]-benzoesäure-isopropylester,
1-(4-Chlor-2-fluor-5-isopropoxyphenyl)-2-methoxy-4-trifluormethyl-6(1H)-pyrimidinon,
1-(4-Chlor-2-fluor-5-isopropoxyphenyl)-2-methoxy-4-pentafluoräthyl-6(1H)-pyrimidinon,
1-(4-Chlor-2-fluor-5-isopropoxyphenyl)-5-fluor-2-methoxy-4-pentafluoräthyl-6(1H)-pyrimidinon,
2-Chlor-4-fluor-5-[2-methoxy-6-oxo-4-trifluormethyl-1-(6H)-pyrimidinyl]-benzoesäure-isopropylester,
2-Chlor-4-fluor-5-[5-fluor-2-methoxy-6-oxo-4-trifluormethyl-1(6H)-pyrimidinyl]-benzoesäure-isopropylester,
1-(4-Chlor-2-fluor-5-propargyloxyphenyl)-2-methoxy-4-trifluormethyl-6(1H)-pyrimidinon,
2-Chlor-4-fluor-5-[2-methoxy-6-oxo-4-pentafluoräthyl-1(6H)-pyrimidinyl]-benzoesäure-methylester,
2-Chlor-4-fluor-5-[2-methoxy-6-oxo-4-pentafluoräthyl-1(6H)-pyrimidinyl]-benzoesäure-äthylester,
2-Chlor-4-fluor-5-[2-methoxy-6-oxo-4-pentafluoräthyl-1(6H)-pyrimidinyl]-benzoesäure-(n-propyl)ester und
2-Chlor-4-fluor-5-[2-methoxy-6-oxo-4-pentafluoräthyl-1(6H)-pyrimidinyl]-benzoesäure-(n-butyl)ester

ausgewählten Verbindung sowie Formulierungshilfsstoffe enthält.

18. Unkrautbekämpfungsmittel nach Anspruch 15, dadurch gekennzeichnet, dass es eine wirksame Menge von 2-Chlor-4-fluor-5-[2-methoxy -6-oxo-4-pentafluoräthyl-1(6H)-pyrimidinyl]-benzoesäure -2-methoxyäthylester sowie Formulierungshilfsstoffe enthält.

19. Unkrautbekämpfungsmittel, dadurch gekennzeichnet, dass es eine wirksame Menge mindestens einer Verbindung der allgemeinen Formel

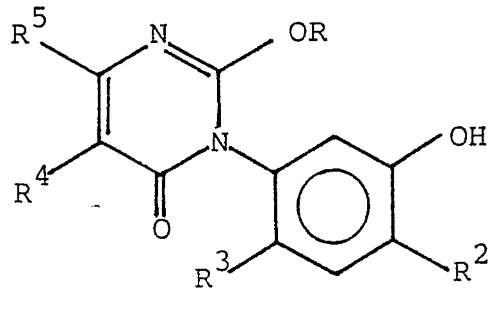

IVa

bzw.

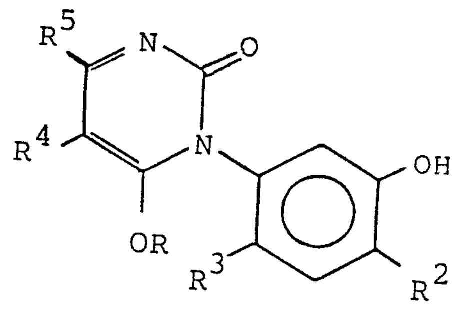

IVb

worin

R $C_{1-4}$-Alkyl, $C_{2-4}$-Alkenyl oder $C_{3\ \text{oder}\ 4}$-Alkinyl,
$R^2$ Halogen oder Cyano,
$R^3$ Wasserstoff oder Halogen,
$R^4$ Wasserstoff, Fluor oder $C_{1-4}$-Alkyl und
$R^5$ $C_{1-4}$-Alkyl oder $C_{1-4}$-Halogenalkyl,

oder

$R^4$ und $R^5$ zuammen Tri-oder Tetramethylen bedeuten,

sowie Formulierungshilfsstoffe enthält.

20. Verfahren zur Herstellung von $C_{1-4}$-Alkyl-, $C_{2-4}$-Alkenyl-und $C_{3\ oder\ 4}$-Alkinyl-enoläthern von 3-Aryluracilen der allgemeinen Formel

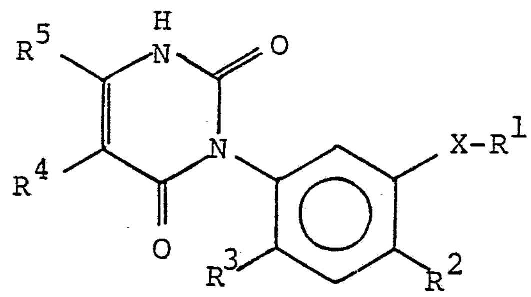

I'

worin

$R^1$ $C_{1-8}$-Alkyl, $C_{2-6}$-Alkenyl, $C_{2-6}$-Alkinyl, $C_{2-8}$-Alkoxyalkyl oder eine Gruppe

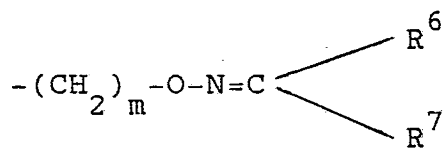

$R^2$ Halogen oder Cyano,

$R^3$ Wasserstoff oder Halogen,

$R^4$ Wasserstoff, Fluor oder $C_{1-4}$-Alkyl,

$R^5$ $C_{1-4}$-Alkyl oder $C_{1-4}$-Halogenalkyl,

oder

$R^4$ und $R^5$ zusammen Tri-oder Tetramethylen,

$R^6$ und $R^7$ unabhängig voneinander $C_{1-4}$-Alkyl,

m 1 oder 2

und

X O, O-C(O), O-C(O)-O oder C(O)-O bedeuten,

dadurch gekennzeichnet dass man

a) ein Uracilderivat der allgemeinen Formel

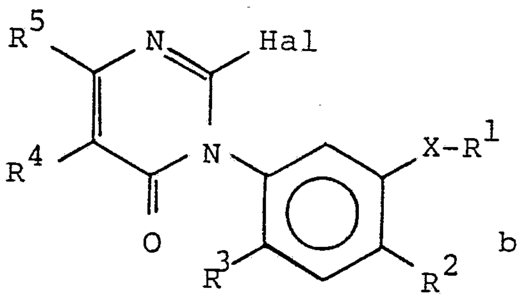

IIa

bzw.

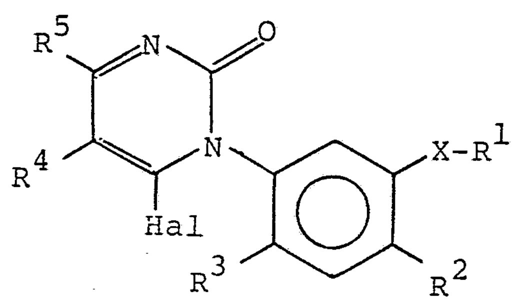

IIb

worin $R^1$-$R^5$ und X die oben angegebenen Bedeutungen besitzen und Hal Chlor oder Brom bedeutet, mit einem Alkohol ROH in Gegenwart einer organischen Base, oder mit dem Metallalkoholat der allgemeinen Formel

$RO^{\ominus}M^{\oplus}$ III

worin R $C_{1-4}$-Alkyl, $C_{2-4}$-Alkenyl oder $C_{3\ oder\ 4}$-Alkinyl und $M^{\oplus}$ ein Aequivalent eines Metallions bedeuten,

behandelt, oder

b) ein 3-Aryluracil der oben angegebenen allgemeinen Formel I', in der X O oder C(O)-O bedeutet, unter basischen Reaktionsbedingungen einer entsprechenden Alkylierung unterwirft, oder

c) in einem Uracilderivat der allgemeinen Formel

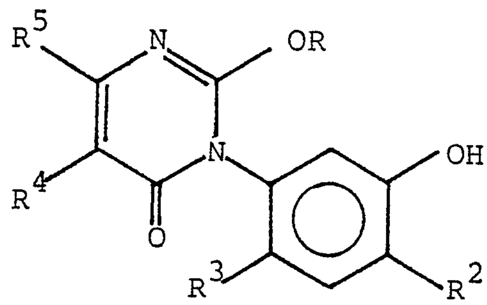

bzw.

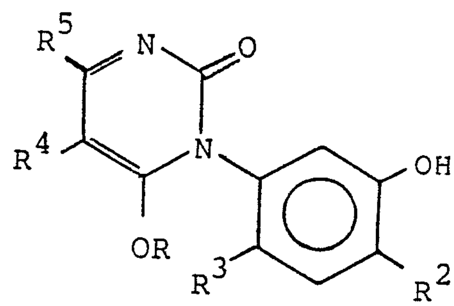

IVa IVb

worin R, $R^2$, $R^3$, $R^4$ und $R^5$ die oben angegebenen Bedeutungen besitzen,

die Hydroxygruppe in an sich bekannter Weise durch den Substituenten X'-$R^1$ ersetzt, worin X' für O, O-C-(O) oder O-C(O)-O steht, oder

d) eine Benzoesäure der allgemeinen Formel

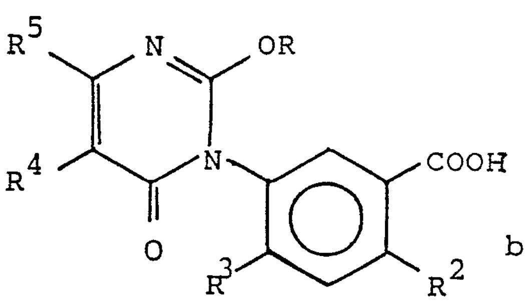

bzw.

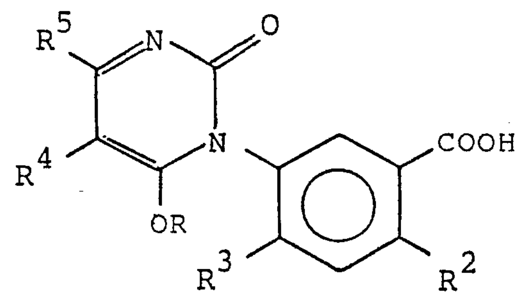

Va Vb

worin R, $R^2$, $R^3$, $R^4$ und $R^5$ die oben angegebenen Bedeutungen besitzen,

oder ein reaktionsfähiges Derivat davon entsprechend verestert,

und dass man den gewünschten Enoläther isoliert.

21. Verfahren nach Anspruch 20, dadurch gekennzeichnet, dass man $C_{1-4}$-Alkyl-, $C_{3\ oder\ 4}$-Alkenyl-und $C_{3\ oder\ 4}$-Alkinyl-enoläther von 3-Aryluracilen der Formel I', worin $R^1$ $C_{1-6}$ -Alkyl, $C_{3-6}$-Alkenyl, $C_{3-6}$-Alkinyl, $C_{2-7}$-Alkoxyalkyl oder eine Gruppe

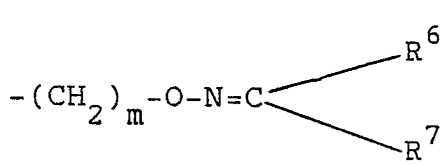

und $R^2$ Halogen bedeuten, herstellt, indem man

a) ein Uracilderivat der allgemeinen Formel

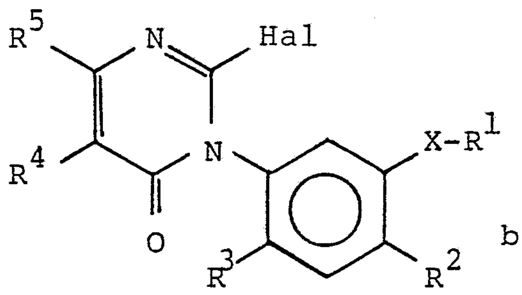

bzw.

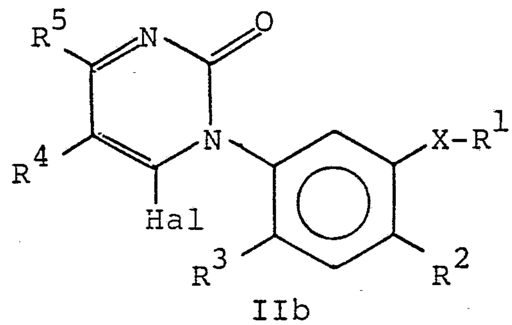

IIa IIb

worin $R^1$-$R^5$ und X die in Anspruch 20 angegebenen Bedeutungen besitzen und Hal Chlor oder Brom bedeutet,

mit einem Metallalkoholat der allgemeinen Formel

$RO^{\ominus}M^{\oplus}$ III

worin R $C_{1-4}$-Alkyl, $C_{3\ oder\ 4}$-Alkenyl oder $C_{3\ oder\ 4}$-Alkinyl und $M^{\oplus}$ ein Aequivalent eines Metallions bedeuten,

behandelt, oder

b) ein 3-Aryluracil der in Anspruch 20 angegebenen allgemeinen Formel I', in der X O oder C(O)-O bedeutet, unter basischen Reaktionsbedingungen einer entsprechenden Alkylierung unterwirft, oder

c) in einem Uracilderivat der allgemeinen Formel

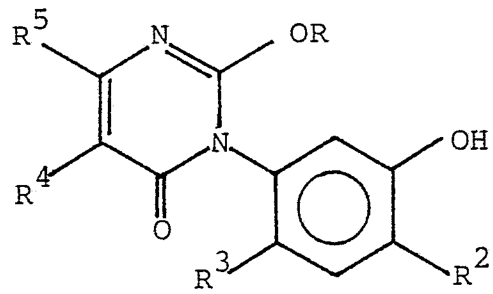

IVa

worin R, $R^2$, $R^3$, $R^4$ und $R^5$ die in Anspruch 20 angegebenen Bedeutungen besitzen,

die Hydroxygruppe in an sich bekannter Weise durch den Substituenten X'-$R^1$ ersetzt, worin X' für O, O-C(O) oder O-C(O)-O steht.

22. Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 1 bis 3, 8, 10, 11 und 13 bzw. eines Mittels gemäss einem der Ansprüche 15, 18 und 19 behandelt.

23. Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 4 bis 7, 9, 12 und 14 bzw. eines Mittels gemäss Anspruch 16 oder 17 behandelt.

24. Verwendung einer Verbindung gemäss einem der Ansprüche 1 bis 3, 8, 10, 11 und 13 bzw. eines Mittels gemäss einem der Ansprüche 15, 18 und 19 zur Bekämpfung von Unkräutern.

25. Verwendung einer Verbindung gemäss einem der Ansprüche 4 bis 7, 9, 12 und 14 bzw. eines Mittels gemäss Anspruch 16 oder 17 zur Bekämpfung von Unkräutern.

Patentansprüche für den folgenden Vertragstaat : ES

1. Verfahren zur Herstellung von $C_{1-4}$-Alkyl-, C $_{2-4}$-Alkenyl-und $C_3$ $_{oder}$ $_4$-Alkinyl-enoläthern von 3-Aryluracilen der allgemeinen Formel

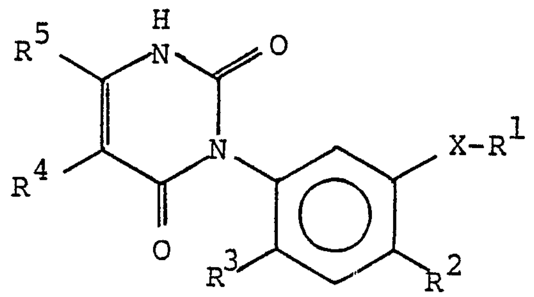

I'

worin

$R^1$ $C_{1-8}$-Alkyl, $C_{2-6}$-Alkenyl, C $_{2-6}$-Alkinyl, $C_{2-8}$-Alkoxyalkyl oder eine Gruppe

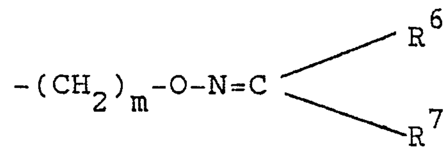

$R^2$ Halogen oder Cyano,

$R^3$ Wasserstoff oder Halogen,

$R^4$ Wasserstoff, Fluor oder $C_{1-4}$-Alkyl,

$R^5$ $C_{1-4}$-Alkyl oder C $_{1-4}$-Halogenalkyl,

oder

$R^4$ und $R^5$ zusammen Tri-oder Tetramethylen,

$R^6$ und $R^7$ unabhängig voneinander $C_{1-4}$-Alkyl,

m 1 oder 2

und

X O, O-C(O), O-C(O)-O oder C(O)-O bedeuten.

dadurch gekennzeichnet dass man

a) ein Uracilderivat der allgemeinen Formel

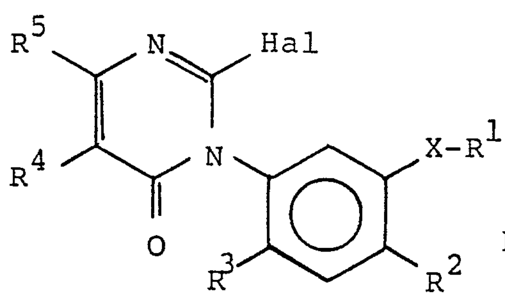

IIa

bzw.

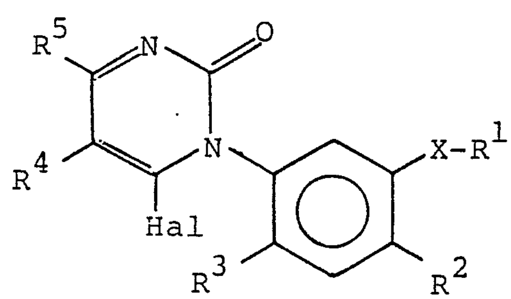

IIb

worin $R^1$-$R^5$ und X die oben angegebenen Bedeutungen besitzen und Hal Chlor oder Brom bedeutet, mit einem Alkohol ROH in Gegenwart einer organischen Base, oder mit dem Metallalkoholat der allgemeinen Formel

$RO^{\ominus}M^{\oplus}$ III

worin R $C_{1-4}$-Alkyl, $C_{2-4}$-Alkenyl oder $C_3$ $_{oder}$ $_4$-Alkinyl und $M^{\oplus}$ ein Aequivalent eines Metallions

bedeuten,
behandelt, oder

b) ein 3-Aryluracil der oben angegebenen allgemeinen Formel I', in der X O oder C(O)-O bedeutet, unter basischen Reaktionsbedingungen einer entsprechenden Alkylierung unterwirft, oder

c) in einem Uracilderivat der allgemeinen Formel

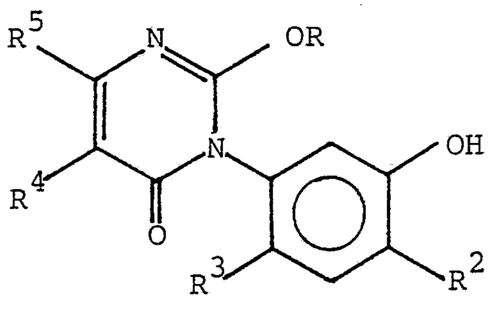

bzw

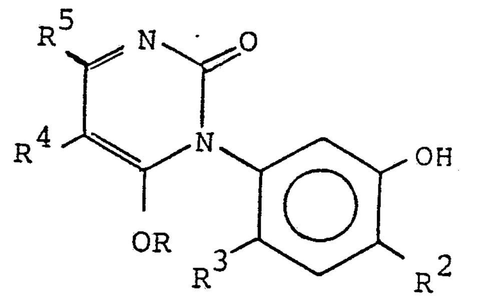

IVa IVb

worin R, $R^2$, $R^3$, $R^4$ und $R^5$ die oben angegebenen Bedeutungen besitzen, die Hydroxygruppe in an sich bekannter Weise durch den Substituenten $X'-R^1$ ersetzt, worin X' für O, O-C-(O) oder O-C(O)-O steht, oder

d) eine Benzoesäure der allgemeinen Formel

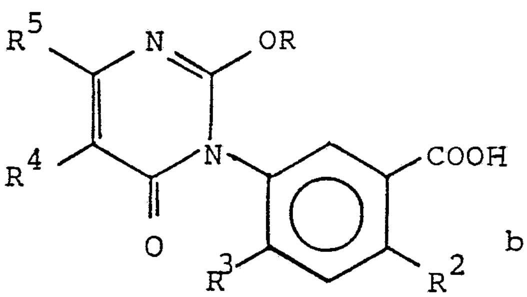

bzw.

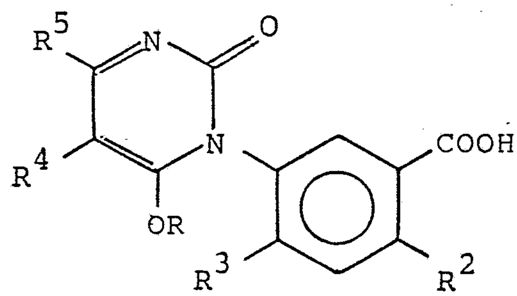

Va Vb

worin R, $R^2$, $R^3$, $R^4$ und $R^5$ die oben angegebenen Bedeutungen besitzen, oder ein reaktionsfähiges Derivat davon entsprechend verestert,

und dass man den gewünschten Enoläther isoliert.

2. Verfahren nach Anspruch 1, dadurch gekennzeichnet, dass man $C_{1-4}$-Alkyl-, $C_{3\ oder\ 4}$-Alkenyl-und $C_{3\ oder\ 4}$-Alkinyl-enoläther von 3-Aryluracilen der Formel I', worin $R^1$ $C_{1-6}$ -Alkyl, $C_{3-6}$-Alkenyl, $C_{3-6}$-Alkinyl, $C_{2-7}$-Alkoxyalkyl oder eine Gruppe

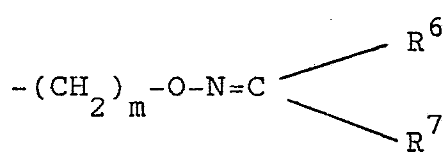

und $R^2$ Halogen bedeuten, herstellt, indem man

a) ein Uracilderivat der allgemeinen Formel

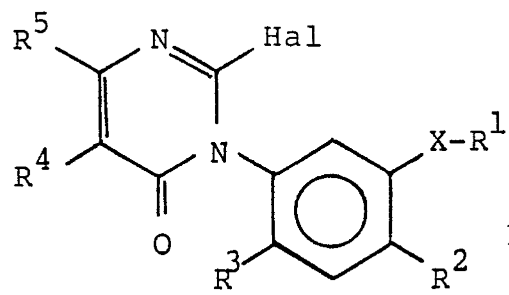

bzw.

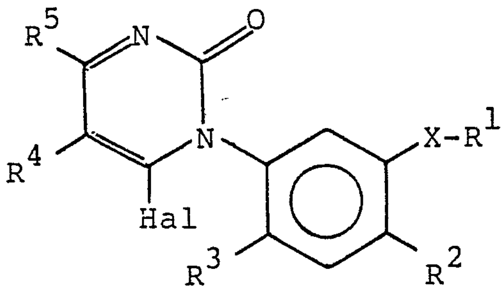

IIa IIb

worin $R^1$-$R^5$ und X die in Anspruch 1 angegebenen Bedeutungen besitzen und Hal Chlor oder Brom bedeutet,

mit einem Metallalkoholat der allgemeinen Formel

$RO^{\ominus}M^{\oplus}$ III

worin R $C_{1-4}$-Alkyl, $C_{3\ \mathrm{oder}\ 4}$-Alkenyl oder $C_{3\ \mathrm{oder}\ 4}$-Alkinyl und $M^{\oplus}$ ein Aequivalent eines Metallions bedeuten,

behandelt, oder

b) ein 3-Aryluracil der in Anspruch 1 angegebenen allgemeinen Formel I', in der X O oder C(O)-O bedeutet, unter basischen Reaktionsbedingungen einer entsprechenden Alkylierung unterwirft, oder

c) in einem Uracilderivat der allgemeinen Formel

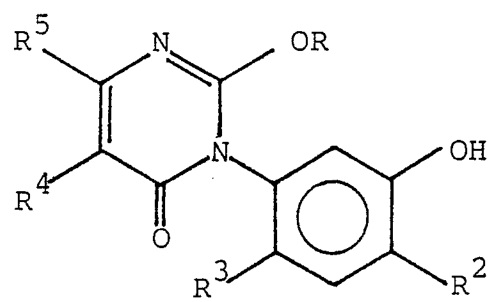

IVa

worin R, $R^2$, $R^3$, $R^4$ und $R^5$ die in Anspruch 1 angegebenen Bedeutungen besitzen,

die Hydroxygruppe in an sich bekannter Weise durch den Substituenten X'-$R^1$ ersetzt, worin X' für O, O-C-(O) oder O-C(O)-O steht.

3. Verfahren nach Anspruch 1 oder 2, worin $R^2$ Chlor oder Brom bedeutet.

4. Verfahren nach einem der Ansprüche 1 bis 3, worin $R^3$ Fluor bedeutet.

5. Verfahren nach einem der Ansprüche 1 bis 4, worin $R^5$ Trifluormethyl bedeutet.

6. Verfahren nach einem der Ansprüche 1 bis 4, worin $R^5$ Pentafluoräthyl bedeutet.

7. Verfahren nach Anspruch 1 zur Herstellung einer Verbindung ausgewählt aus:

2-Chlor-4-fluor-5-[2-methoxy-6-oxo-4-pentafluoräthyl-1(6H)-pyrimidinyl]-benzoesäure-isopropylester,

2-Chlor-4-fluor-5-[5-fluor-2-methoxy-6-oxo-4-pentafluoräthyl-1(6H)-pyrimidinyl]-benzoesäure-isopropylester,

1-(4-Chlor-2-fluor-5-isopropoxyphenyl)-2-methoxy-4-trifluormethyl-6(1H)-pyrimidinon,

1-(4-Chlor-2-fluor-5-isopropoxyphenyl)-2-methoxy-4-pentafluoräthyl-6(1H)-pyrimidinon,

1-(4-Chlor-2-fluor-5-isopropoxyphenyl)-5-fluor-2-methoxy-4-pentafluoräthyl-6(1H)-pyrimidinon,

2-Chlor-4-fluor-5-[2-methoxy-6-oxo-4-trifluormethyl-1(6H)-pyrimidinyl]-benzoesäure-isopropylester,

2-Chlor-4-fluor-5-[5-fluor-2-methoxy-6-oxo-4-trifluormethyl-1(6H)-pyrimidinyl]-benzoesäure-isopropylester,

1-(4-Chlor-2-fluor-5-propargyloxyphenyl)-2-methoxy-4-trifluormethyl-6(1H)-pyrimidinon.

2-Chlor-4-fluor-5-[2-methoxy-6-oxo-4-pentafluoräthyl-1(6H)-pyrimidinyl]-benzoesäure-methylester,

2-Chlor-4-fluor-5-[2-methoxy-6-oxo-4-pentafluoräthyl-1(6H)-pyrimidinyl]-benzoesäure-äthylester, 2-Chlor-4-fluor-5-[2-methoxy-6-oxo-4-pentafluoräthyl-1(6H)-pyrimidinyl]-benzoesäure-(n-propyl)ester und

2-Chlor-4-fluor-5-[2-methoxy-6-oxo-4-pentafluoräthyl-1(6H)-pyrimidinyl]-benzoesäure-(n-butyl)ester.

8. Verfahren nach Anspruch 1 zur Herstellung von 2-Chlor-4-fluor-5-[2-methoxy-6-oxo-4-pentafluoräthyl-1(6H)-pyrimidinyl]-benzoesäure-2-methoxyäthylester.

9. Verfahren zur Herstellung von Verbindungen der allgemeinen Formel

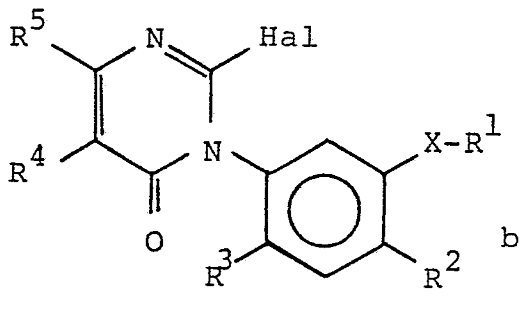

bzw.

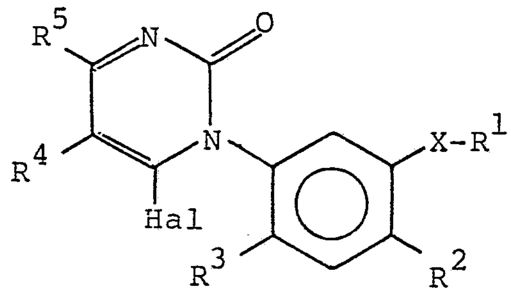

IIa IIb

worin

$R^1$ $C_{1-8}$-Alkyl, $C_{2-6}$-Alkenyl, $C_{2-6}$-Alkinyl, $C_{2-8}$-Alkoxyalkyl oder eine Gruppe

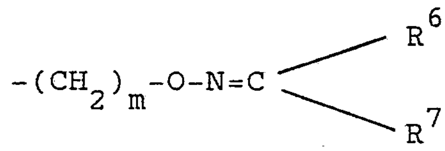

$R^2$ Halogen oder Cyano,
$R^3$ Wasserstoff oder Halogen,
$R^4$ Wasserstoff, Fluor oder $C_{1-4}$-Alkyl,
$R^5$ $C_{1-4}$-Alkyl oder $C_{1-4}$-Halogenalkyl,
oder
$R^4$ und $R^5$ zusammen Tri-oder Tetramethylen,
$R^6$ und $R^7$ unabhängig voneinander $C_{1-4}$-Alkyl,
m 1 oder 2,
X O, O-C(O), O-C(O)-O oder C(O)-O
und
Hal Chlor oder Brom bedeuten,
dadurch gekennzeichnet, dass man ein 3-Aryluracil der allgemeinen Formel

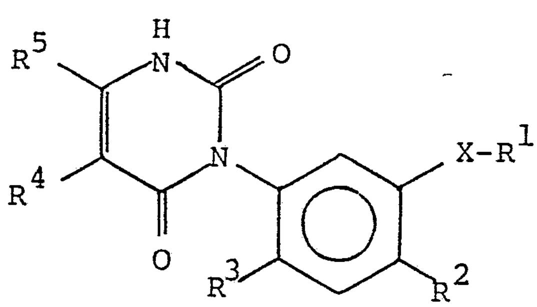

I'

mit einem Chlorierungs-bzw. Bromierungsmittel behandelt.

10. Verfahren nach Anspruch 9, worin $R^1$ $C_{1-6}$-Alkyl, $C_{3-6}$-Alkenyl, $C_{3-6}$-Alkinyl, $C_{2-7}$-Alkoxyalkyl oder eine Gruppe

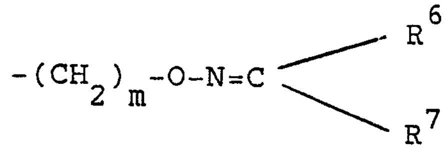

und $R^2$ Halogen bedeuten.

11. Verfahren zur Herstellung von Verbindungen der allgemeinen Formel

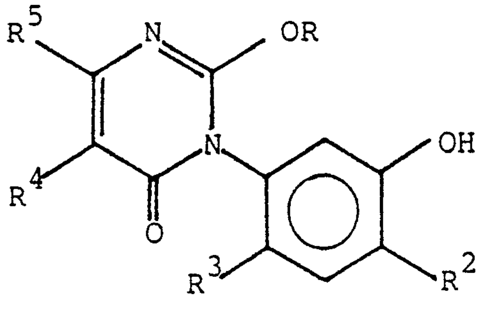

bzw.

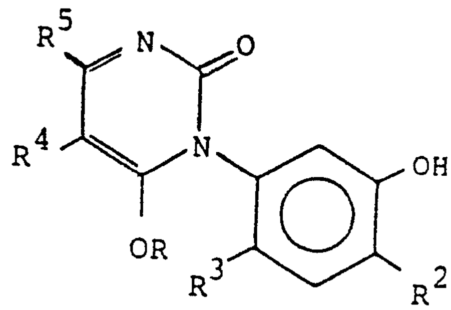

IVa IVb

worin

R $C_{1-4}$-Alkyl, $C_{2-4}$-Alkenyl oder $C_{3\ \text{oder}\ 4}$-Alkinyl,

$R^2$ Halogen oder Cyano,

$R^3$ Wasserstoff oder Halogen,

$R^4$ Wasserstoff, Fluor oder $C_{1-4}$-Alkyl und

$R^5$ $C_{1-4}$-Alkyl oder $C_{1-4}$-Halogenalkyl,

oder

$R^4$ und $R^5$ zuammen Tri-oder Tetramethylen bedeuten,

dadurch gekennzeichnet, dass man das entsprechende geschützte Phenol der allgemeinen Formel

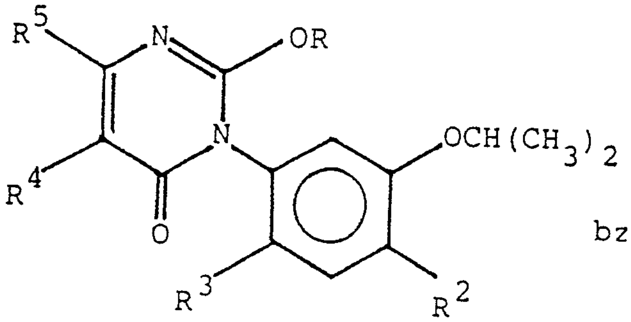

bzw.

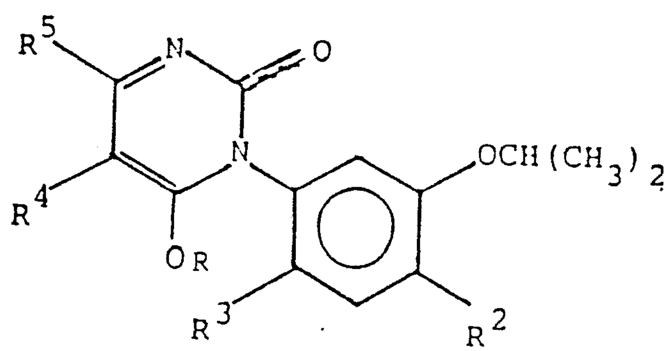

Ia' Ib'

worin R, $R^2$, $R^3$, $R^4$ und $R^5$ die oben angegebenen Bedeutungen besitzen,
einer säurekatalysierten Hydrolyse unterwirft.

12. Verfahren nach Anspruch 11 zur Herstellung von Verbindungen der Formel IVa, worin R $C_{1-4}$-Alkyl, $C_{3\ \text{oder}\ 4}$-Alkenyl oder $C_{3\ \text{oder}\ 4}$-Alkinyl und $R^2$ Halogen bedeuten.

13. Unkrautbekämpfungsmittel, dadurch gekennzeichnet, dass es eine wirksame Menge mindestens eines $C_{1-4}$-Alkyl-, $C_{2-4}$-Alkenyl-oder $C_{3\ \text{oder}\ 4}$-Alkinyl-enoläthers von 3-Aryluracilen der allgemeinen Formel

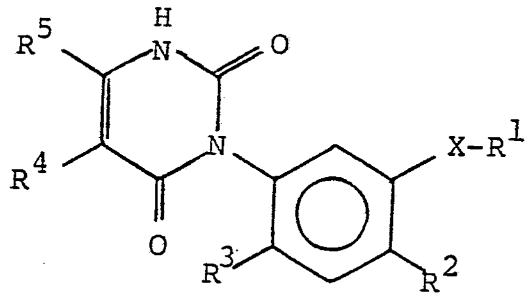

I'

worin

$R^1$ $C_{1-8}$-Alkyl, $C_{2-6}$-Alkenyl, $C_{2-6}$-Alkinyl, $C_{2-8}$-Alkoxyalkyl oder eine Gruppe

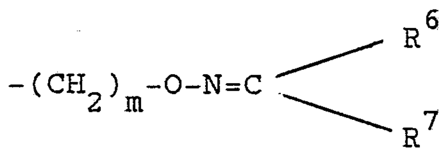

$R^2$ Halogen oder Cyano,
$R^3$ Wasserstoff oder Halogen,
$R^4$ Wasserstoff, Fluor oder $C_{1-4}$-Alkyl,
$R^5$ $C_{1-4}$-Alkyl oder $C_{1-4}$-Halogenalkyl,
oder
$R^4$ und $R^5$ zusammen Tri-oder Tetramethylen,
$R^6$ und $R^7$ unabhängig voneinander $C_{1-4}$-Alkyl,
m 1 oder 2
und
X O, O-C(O), O-C(O)-O oder C(O)-O bedeuten,
sowie Formulierungshilfsstoffe enthält.

14. Unkrautbekämpfungsmittel nach Anspruch 13, dadurch gekennzeichnet, dass es eine wirksame Menge mindestens eines $C_{1-4}$-Alkyl-, $C_{3\ oder\ 4}$-Alkenyl-oder $C_{3\ oder\ 4}$-Alkinyl-enoläthers von 3-Aryluracilen der Formel I', worin $R^1$ $C_{1-6}$-Alkyl, $C_{3-6}$-Alkenyl, $C_{3-6}$-Alkinyl, $C_{2-7}$-Alkoxyalkyl oder eine Gruppe

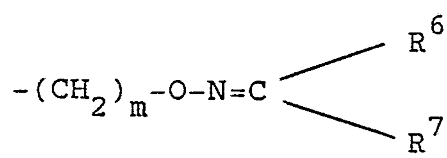

und $R^2$ Halogen bedeuten, sowie Formulierungshilfsstoffe enthält.

15. Unkrautbekämpfungsmittel nach Anspruch 13, dadurch gekennzeichnet, dass es eine wirksame Menge mindestens einer aus der Gruppe

2-Chlor-4-fluor-5-[2-methoxy-6-oxo-4-pentafluoräthyl-1(6H)-pyrimidinyl]-benzoesäure-isopropylester,
2-Chlor-4-fluor-5-[5-fluor-2-methoxy-6-oxo-4-pentafluoräthyl-1(6H)-pyrimidinyl]-benzoesäure-isopropylester,
1-(4-Chlor-2-fluor-5-isopropoxyphenyl)-2-methoxy-4-trifluormethyl-6(1H)-pyrimidinon,
1-(4-Chlor-2-fluor-5-isopropoxyphenyl)-2-methoxy-4-pentafluoräthyl-6(1H)-pyrimidinon,
1-(4-Chlor-2-fluor-5-isopropoxyphenyl)-5-fluor-2-methoxy-4-pentafluoräthyl-6(1H)-pyrimidinon,
2-Chlor-4-fluor-5-[2-methoxy-6-oxo-4-trifluormethyl-1(6H)-pyrimidinyl]-benzoesäure-isopropylester,
2-Chlor-4-fluor-5-[5-fluor-2-methoxy-6-oxo-4-trifluormethyl-1(6H)-pyrimidinyl]-benzoesäure-isopropylester,
1-(4-Chlor-2-fluor-5-propargyloxyphenyl)-2-methoxy-4-trifluormethyl-6(1H)-pyrimidinon,
2-Chlor-4-fluor-5-[2-methoxy-6-oxo-4-pentafluoräthyl-1(6H)-pyrimidinyl]-benzoesäure-methylester,
2-Chlor-4-fluor-5-[2-methoxy-6-oxo-4-pentafluoräthyl-1(6H)-pyrimidinyl]-benzoesäure-äthylester,

2-Chlor-4-fluor-5-[2-methoxy-6-oxo-4-pentafluoräthyl-1(6H)-pyrimidinyl]-benzoesäure-(n-propyl)ester und

2-Chlor-4-fluor-5-[2-methoxy-6-oxo-4-pentafluoräthyl-1(6H)-pyrimidinyl]-benzoesäure-(n-butyl)ester ausgewählten Verbindung sowie Formulierungshilfsstoffe enthält.

16. Unkrautbekämpfungsmittel nach Anspruch 13, dadurch gekennzeichnet, dass es eine wirksame Menge von 2-Chlor-4-fluor-5-[2-methoxy -6-oxo-4-pentafluoräthyl-1(6H)-pyrimidinyl]-benzoesäure -2-methoxyäthlester sowie Formulierungshilfsstoffe enthält.

17. Unkrautbekämpfungsmittel, dadurch gekennzeichnet, dass es eine wirksame Menge mindestens einer Verbindung der allgemeinen Formel

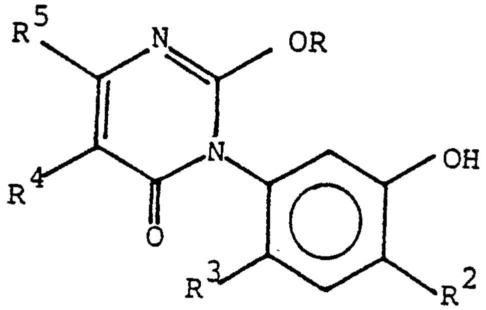

bzw.

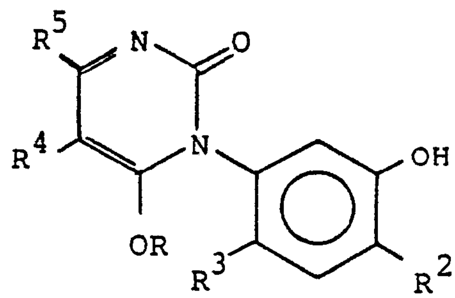

IVa IVb

worin

R $C_{1-4}$-Alkyl, $C_{2-4}$-Alkenyl oder $C_{3\ oder\ 4}$-Alkinyl,
$R^2$ Halogen oder Cyano,
$R^3$ Wasserstoff oder Halogen,
$R^4$ Wasserstoff, Fluor oder $C_{1-4}$-Alkyl und
$R^5$ $C_{1-4}$-Alkyl oder $C_{1-4}$-Halogenalkyl,
oder
$R^4$ und $R^5$ zuammen Tri-oder Tetramethylen bedeuten,
sowie Formulierungshilfsstoffe enthält.

18. Verfahren zur Herstellung eines Unkrautbekämpfungsmittels, dadurch gekennzeichnet, dass man mindestens eine der in Anspruch 1 genannten Enoläther mit Formulierungshilfsstoffen vermischt.